# EUROPEAN PATENT APPLICATION

(11) **EP 2 169 077 A1**
(43) Date of publication of application: **31.03.2010**
(21) Application number: 08164723.2
(22) Date of filing: 19.09.2008
(51) Int. Cl.: C12Q 1/68

(54) **Methods and compositions for diagnosing an adenocarcinoma**

(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: Jungen, Carmen

(57) **Abstract**

The present invention relates to *in vitro* methods and compositions for diagnosing and/or treating an adenocarcinoma associated with a chromosomal aberration on chromosome 20q and/or the predisposition for developing such an adenocarcinoma by determining the expression levels of a set of particular marker genes, wherein an elevated expression level of the marker genes in a test sample as compared to a control level is indicative of an adenocarcinoma.

## Description

### FIELD OF THE INVENTION

The present invention relates to *in vitro* methods and compositions for diagnosing and/or treating an adenocarcinoma associated with a chromosomal aberration on chromosome 20q and/or the predisposition for developing such an adenocarcinoma by determining the expression levels of a set of particular marker genes, wherein an elevated expression level of the marker genes in a test sample as compared to a control level is indicative of an adenocarcinoma.

### BACKGROUND OF THE INVENTION

Most cancers are epithelial in origin and arise through a stepwise progression from normal cells, through dysplasia, into malignant cells that invade surrounding tissues and have metastatic potential. The colorectal adenoma to carcinoma progression is a classic example of this process (Muto, T. et al. (1975) Cancer 36, 2251-2270; Fearon, E.R. and Vogelstein, B. (1990) Cell 61, 759-767). Cancer of the colorectal part of the gastrointestinal tract is a frequently occurring disorder. In a first stage, a benign tumor (i.e. an adenoma) occurs which can turn in to a malignant cancer (adenocarcinoma). However, only a small subset of adenomas progress to adenocarcinomas.

Genomic instability is a crucial step in this progression and occurs in two ways in colorectal cancer (CRC) (Lengauer, C. et al. (1997) Nature 386, 623-627) DNA mismatch repair deficiency leading to microsatellite instability (MIN), explains only about 15% of the cases of adenoma to carcinoma progression (Umar, A. et al. (2004) J. Natl. Cancer Inst. 96, 261-268; di Pietro, M. et al. (2005) Gastroenterology 129, 1047-1059). In the other 85%, genomic instability occurs at the chromosomal level (CIN), giving rise to aneuploidy.

While for a long time chromosomal aberrations have been regarded as random noise, secondary to cancer development, it is now well established that these DNA copy number changes occur in specific patterns and are associated with different clinical behavior (Hermsen, M. et al. (2002) Gastroenterology 123, 1109-1119; Rajagopalan, H. et al. (2003) Nat. Rev. Cancer 3, 695-701). Nevertheless, neither the cause of chromosomal instability in human cancer progression nor its biological consequences have been fully appreciated.

Chromosomal aberrations frequently reported in CRC are 7pq, 8q, 13q, and 20q gains and 4pq, 5q, 8p, 15q, 17p, and 18q losses (Douglas, E.J. et al. (2004) Cancer Res. 64, 4817-4825). Of these, especially 8q, 13q and 20q gains and 8p, 15q, 17p and 18q losses are associated with colorectal adenoma to carcinoma progression.

Gain of 20q is observed in more than 65% of CRCs (De Angelis, P.M. et al. (1999) Br. J. Cancer 80, 526-535). Gains of 20q are also common in other tumor types and have been associated with poor outcome in gastric and CRC. The 20q13 amplicon has been studied in detail in breast and gastric cancers with restricted contig array CGH, pinpointing several genes as targets of amplification (Albertson, D.G. et al. (2000) Nat. Genet. 25, 144-146; Weiss, M.M. et al. (2003) J. Pathol. 200, 320-326). Analysis of DNA copy number changes at gene level by multiplex ligation-dependent probe amplification (MLPA) showed that in CRC, besides 20q13, also 20q11 is frequently amplified (Postma, C. et al. (2005) J. Pathol. 205, 514-521).

However, no gene markers (i.e. oncogenes) have been identified so far that are specifically linked to a given chromosomal aberration associated with CRC and thus allow for diagnosing an adenocarcinoma associated with a particular type of chromosomal aberration and/or the progression of an adenomas to such an adenocarcinomas. The identification of such gene markers would be of utmost clinical importance, particularly if these gene markers enable a reliable diagnosis at an early stage of tumor progression in order to allow early stage treatment of carcinomas while avoiding unnecessary surgical intervention. Ideally, such marker genes should enable the identification of adenocarcinomas at a stage where the presence of malignant cells is not yet detectable by *in situ* techniques or microscopic analysis of biopsy or resection material.

Thus, it is a continuing need for the identification of gene markers and the provision of corresponding methods and compositions using said gene markers for the reliable and accurate diagnosis and/or treatment of an adenocarcinoma associated with a particular type of chromosomal aberration.

### OBJECT AND SUMMARY OF THE INVENTION

It is an objective of the present invention to provide novel approaches for diagnosing and/or treating an adenocarcinoma associated with a chromosomal aberration on chromosome 20q and/or the predisposition for developing such an adenocarcinoma by determining the expression levels of a set of particular marker genes, wherein an elevated expression level of the marker genes in a test sample as compared to a control level is indicative of an adenocarcinoma associated with a chromosomal aberration on chromosome 20q.

More specifically, it is an object of the present invention to provide methods and compositions for diagnosing the progression from an adenoma to an adenocarcinoma, that is, for discriminating between benign and malignant tumors.

These objectives as well as others, which will become apparent from the ensuing description, are attained by the subject matter of the independent claims. Some of the preferred embodiments of the present invention are defined by the subject matter of the dependent claims.

In one aspect, the present invention relates to *an in vitro* method for diagnosing in a subject an adenocarcinoma associated with a chromosomal aberration on chromosome 20q, the method comprising the steps of: (a) detecting in a test sample obtained from the subject the expression level(s) of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397); and (b) comparing the expression level(s) obtained in step (a) to a control level, wherein an elevated expression level of any one of the marker genes in the test sample as compared to the control level is indicative of an adenocarcinoma associated with a chromosomal aberration on chromosome 20q in the subject.

In specific embodiments, the method is for the further use of diagnosing a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q.

Preferably, the chromosomal aberration on chromosome 20q is an aberration located at position 20q11.22-20q11.23 and/or at position 20q13.31-20q13.33. Particularly preferably, the chromosomal aberration is a chromosomal gain.

In a further preferred embodiment of the method, the expression levels of at least the marker genes *RNPC1* (Genbank accession # NM_017495) and *TCFL5* (Genbank accession # NM_006602) are detected, wherein elevated expression levels of both said marker genes in the test sample as compared to the control level are indicative of an adenocarcinoma, a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q in the subject.

The expression level(s) of the marker gene(s) may be determined by any one or more of the methods selected from the group consisting of: (a) detecting a mRNA encoded by the marker gene(s); (b) detecting a protein encoded by the marker gene(s); and (c) detecting a biological activity of a protein encoded by the marker gene(s).

In a further preferred embodiment, the method further comprises a step (c) of detecting a chromosomal aberration on chromosome 20q, preferably by comparative genomic hybridization (CGH), PCR detection or multiplex ligation-dependent probe amplification (MPLA).

In a further aspect the present invention relates to *an in vitro* method for diagnosing in a subject an adenocarcinoma, the method comprising: (a) detecting in a test sample obtained from the subject a chromosomal gain on chromosome 20q; and in case a chromosomal gain is detected on chromosome 20q further comprising the steps of (b) detecting in said sample the expression level(s) of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397); and (c) comparing the expression level(s) obtained in step (b) to a control level, wherein an elevated expression level of any one of the marker genes in the test sample as compared to the control level is indicative of an adenocarcinoma.

In another preferred embodiment, the present invention relates to an *in vitro* method for diagnosing in a subject an adenocarcinoma comprising the detection of a chromosomal gain on chromosome 20q as described above, wherein the detection of said chromosomal gain on chromosome 20q is performed by comparative genomic hybridization (CGH), PCR detection or multiplex ligation-dependent probe amplification (MPLA).

In another aspect, the present invention relates to a kit for diagnosing an adenocarcinoma comprising means for detecting the expression of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397).

Preferably, the kit further comprises means for detecting a chromosomal aberration on chromosome 20q.

In yet another aspect, the present invention relates to a method of identifying an agent for treating or preventing adenocarcinoma, the method comprising the steps of: (a) contacting a test agent with one or more cells expressing any one or more of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397); (b) detecting the expression level(s) of the one or more marker genes; and (c) selecting a test agent that reduces the expression level(s) of any one or more of the marker gene as compared to that (those) detected in the absence of the test agent.

In a further aspect, the present invention relates to a pharmaceutical composition comprising any one or more agents selected from the group consisting of: an antisense nucleic acid construct, an siRNA, a riboyzme or an antibody directed against or a dominant negative polypeptide variant of any one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397).

Preferably, the pharmaceutical composition is employed for the prevention and/or treatment of an adenocarcinoma.

In yet another aspect, the present invention relates to the use of an antisense nucleic acid construct, an siRNA, a riboyzme or an antibody directed against or a dominant negative polypeptide variant of any one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397) for the preparation of a pharmaceutical composition for the prevention and/or treatment of an adenocarcinoma.

Other embodiments of the present invention will become apparent from the detailed description hereinafter.

### DESCRIPTON OF THE FIGURES

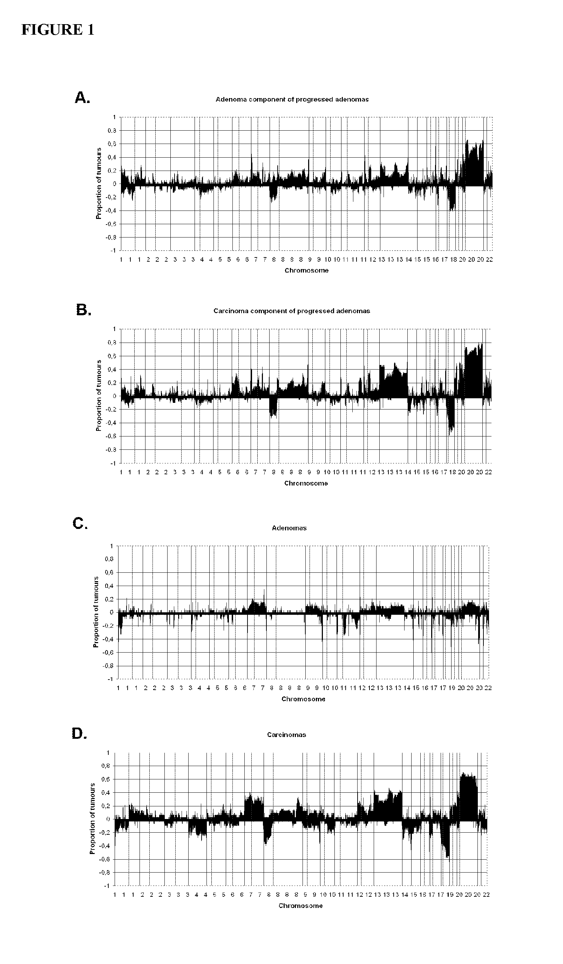

- Fig. 1: shows a frequency plot of DNA copy number gains and losses as determined by BAC array comparative genomic hybridization in: (A) adenoma components of 41 progressed colorectal adenomas, (B) adenocarcinoma components of 41 progressed colorectal adenomas, (C) 34 non-progressed colorectal adenomas, and (D) 33 adenocarcinomas. Y-axis displays the fraction of tumors with either a gain (positive sign) or loss (negative sign) for all clones that are sorted by chromosome and base pair position.

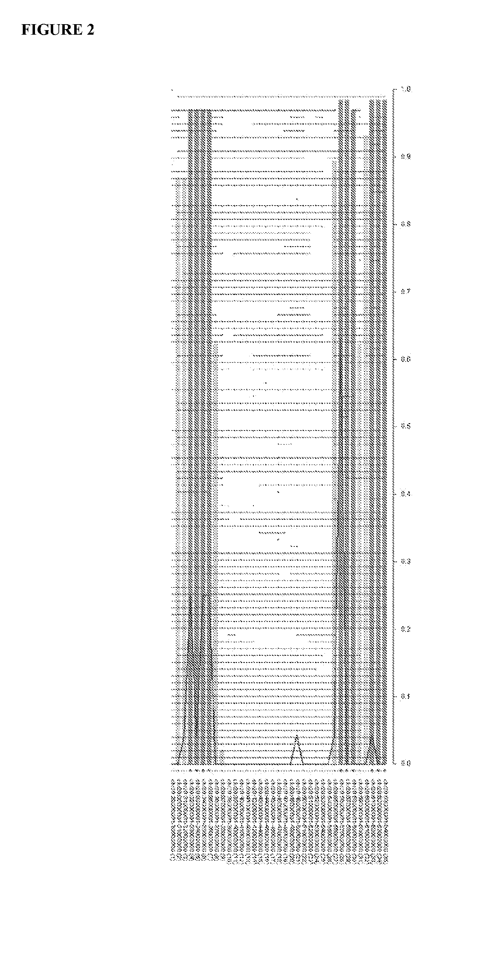

- Fig. 2: depicts the delimitation of the smallest regions of overlap (SROs) by STAC analysis for 115 samples (41 non-progressed adenomas, 41 adenocarcinoma components of progressed adenomas, and 33 adenocarcinomas). Results for the long arm of chromosome 20 are displayed. Rows represent samples, and columns represent chromosomal locations. A black dot indicates a gain called in a sample at a location. Consecutive black dots are connected via a line to represent an interval of aberration. Grey bars track the maximum STAC confidence (1 - P-value), darker bars are those with confidence of > 0.95. The line graph indicates the actual frequencies in the sample set.

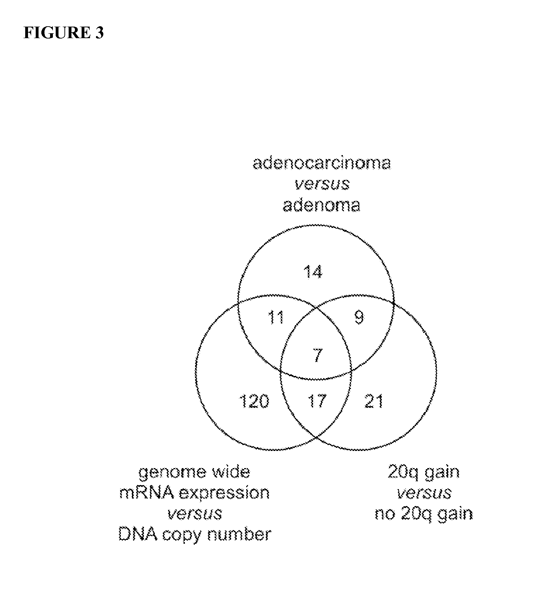

- Fig. 3: shows a Venn diagram integrating results of three different data analysis approaches (comparing colorectal adenocarcinomas *versus* adenomas; colorectal tumors with a 20q gain *versus* tumors without a 20q gain; and genome wide integration of mRNA expression data with DNA copy number data). Seven genes (*C20orf24, AURKA, RNPC1, TH1L, ADRM1, C20orf20*, and *TCFL5*) emerge with all three approaches.

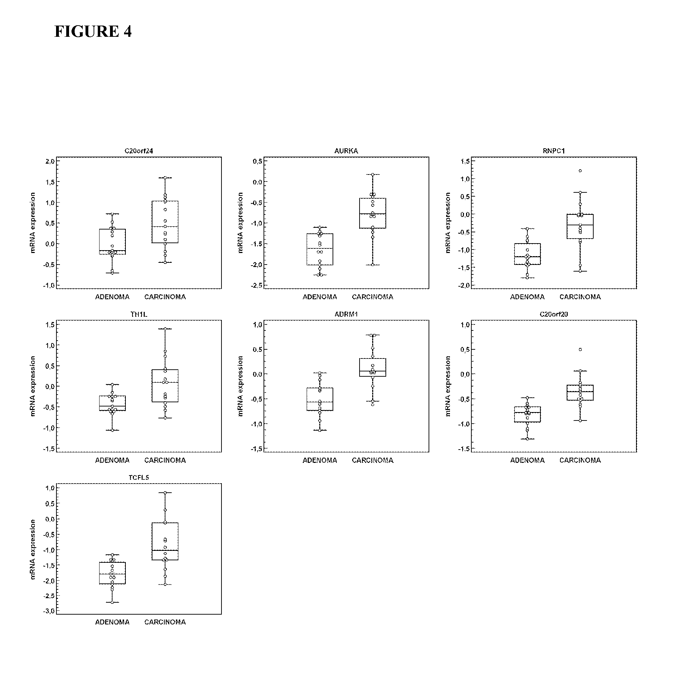

- Fig. 4: depicts the integration of expression microarray data and array CGH data of genes *C20orf24, AURKA, RNPC1, TH1L, ADRM1, C20orf20*, and *TCFL5*. Combined box plots with dot plots of mRNA expression (determined by oligonucleotide microarrays) in colorectal adenomas and carcinomas.

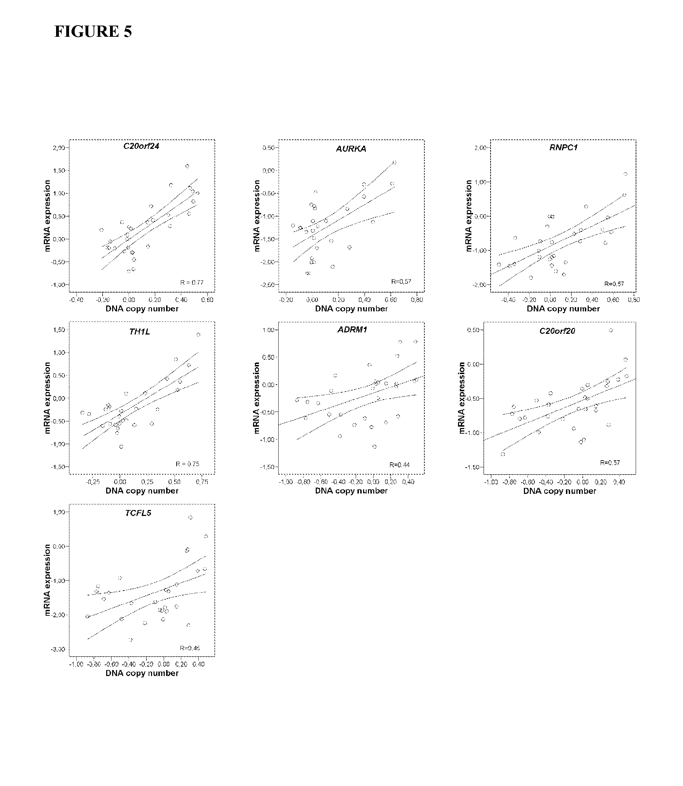

- Fig. 5: depicts the integration of expression microarray data and array CGH data of genes *C20orf24, AURKA, RNPC1, TH1L, ADRM1, C20orf20*, and *TCFL5*. Scatter plots showing correlation of mRNA expression (determined by oligonucleotide microarrays) and DNA copy number (determined by BAC array CGH).

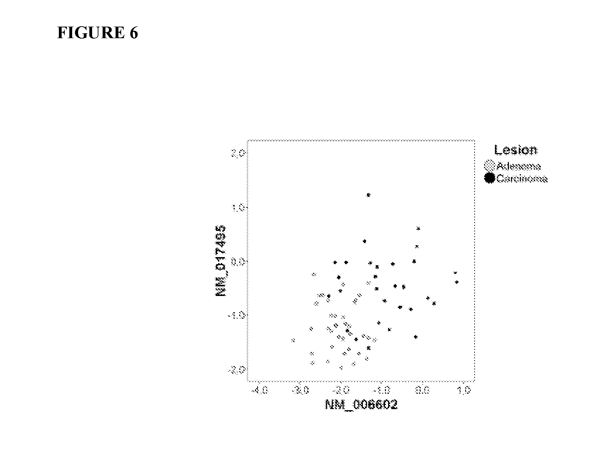

- Fig. 6: shows a scatter plot of mRNA expression levels of *RNPC1* (Genbank accession # NM_017495) and *TCFL5* (Genbank accession # NM_006602), by lesion (grey circles: adenomas; black circles: carcinomas) showing a good separation of colorectal adenomas *versus* adenocarcinomas.

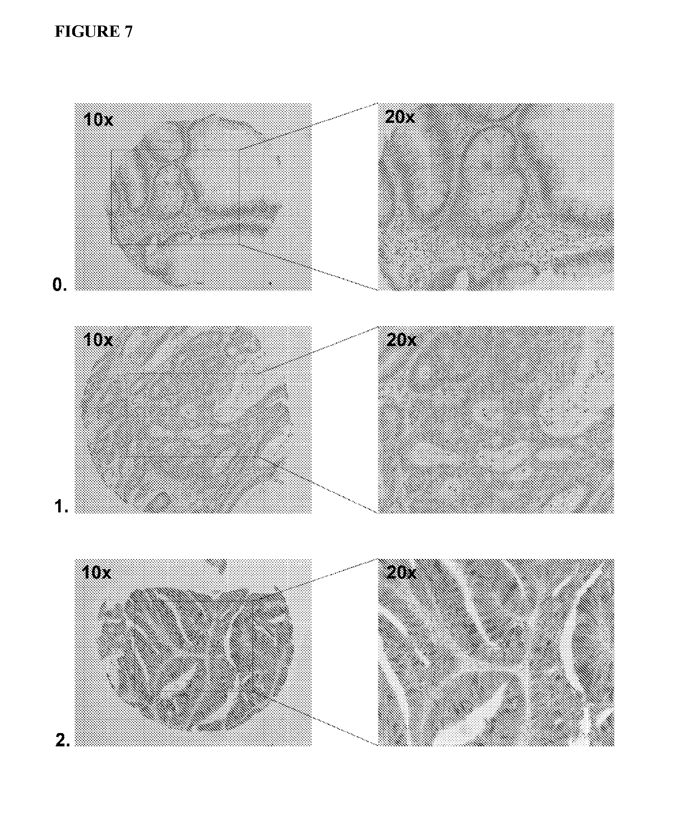

- Fig. 7: shows examples of AURKA protein expression in TMA cores of an adenoma showing no expression (0), an adenocarcinoma showing weak expression (1), and an adenocarcinoma showing strong expression (2).

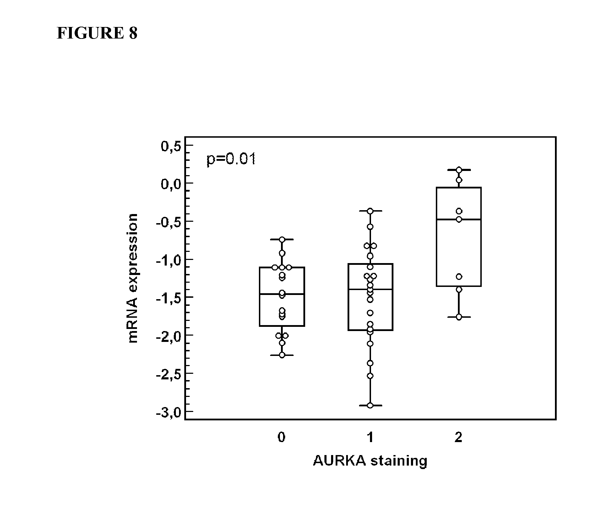

- Fig. 8: depicts a combined box plot with dot plot of mRNA expression, determined by oligonucleotide microarrays (Y-axis), of colorectal adenomas and carcinomas with a negative (0), weak (1) or strong (2) protein expression of AURKA on immunohistochemistry (X-axis).

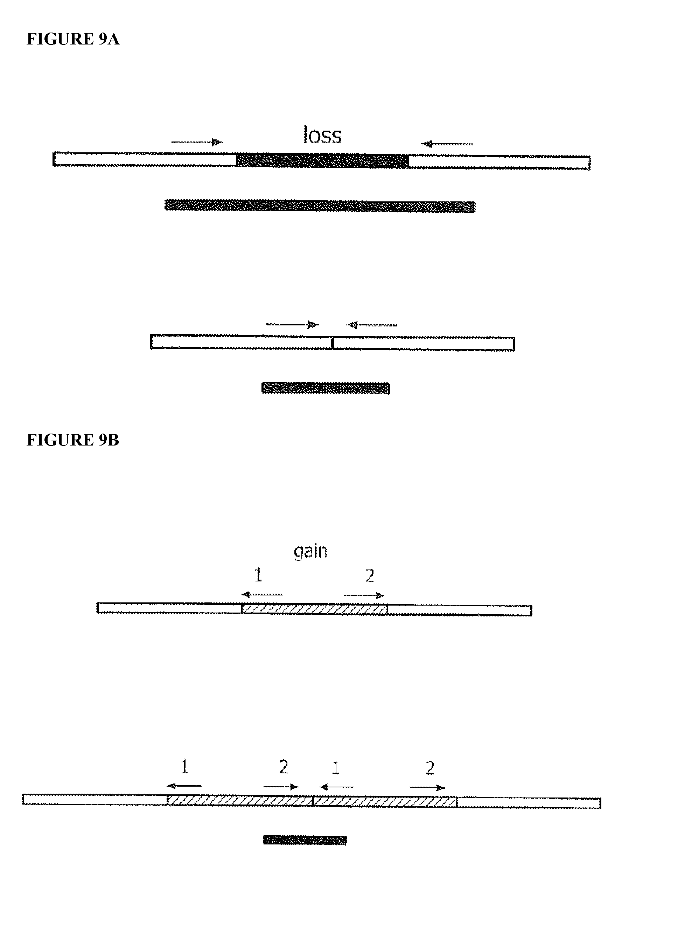

- Fig. 9: schematically illustrates the principle of detecting chromosomal loss (A) or gain (B) in a polynucleotide sequence using a qualitative PCR reaction. The figure shows a part of genomic DNA before and after the chromosomal aberration. Arrows represent PCR primers. The length of the PCR fragments (if generated) is shown below the genomic DNA.

### DETAILED DESCRIPTION OF THE INVENTION

The present invention is based on the unexpected finding that the detection of an elevated expression of any one or more of only seven specific marker genes in a test sample of a subject as compared to a control level allows for diagnosing an adenocarcinoma associated with a chromosomal aberration on chromosome 20q, i.e. a particular type of adenocarcinoma, and/or a predisposition for developing such an adenocarcinoma with high accuracy and reliability.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are nonlimiting. In the drawings, the size of some of the elements may be exaggerated and not drawn on scale for illustrative purposes.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

The following terms or definitions are provided solely to aid in the understanding of the invention. These definitions should not be construed to have a scope less than understood by a person of ordinary skill in the art.

The term "tumor" or "neoplasm", as used herein, refers to an abnormal tissue that grows by cellular proliferation more rapidly than normally, and continues to grow after the stimuli that initiated the new growth cease. The term "lesion", generally referring to an abnormality involving any tissue or organ due to any disease or any injury, is also used herein to refer to a neoplasm. Tumors, neoplasm or lesions can be either benign or malignant.

The term "cancer", as used herein, is a general term referring to any type of malignant neoplasm.

The term "adenocarcinoma", as used herein, relates to a malignant neoplasm of epithelial cells. Typically, adenocarcinoma is a cancer that originates in glandular tissue. This tissue is part of a more general type of tissue known as epithelial tissue. Epithelial tissue includes skin, glands and a variety of other tissue lining/surrounding the cavities and organs of the body. Embryologically, the epithelium is derived from ectoderm, endoderm and mesoderm. In order to be classified as adenocarcinoma, the cells do not necessarily need to be part of a gland, as long as they have secretory properties. Hence, adenocarcinomas are also often referred to as "glandular cancer" or "glandular carcinoma". An adenocarcinoma can occur in some higher mammals, including humans. Highly differentiated adenocarcinomas tend to resemble the glandular tissue that they are derived from, while poorly differentiated may not. Traditionally, a pathologist could verify whether a tumor is an adenocarcinoma or some other type of cancer determine by staining the cells from a biopsy. Such an independent examination may be used as additional means of diagnosis or diagnostic verification once a diagnoses has been obtained according to the method(s) of the present invention.

Adenocarcinomas can arise in many tissues of the body due to the ubiquitous nature of glands within the body. While each gland may not be secreting the same substance, as long as there is an exocrine function to the cell, it is considered glandular and its malignant form is therefore named adenocarcinoma. However, endocrine gland tumors, such as a VIPoma, an insulinoma, a pheochromocytoma, etc., are typically not referred to as adenocarcinomas but rather are often designated neuroendocrine tumors. Nonetheless, for the purpose of the present invention, also the diagnosis of these tumor types is to be understood as comprised in a specific embodiment of the present invention.

If the glandular tissue is abnormal, but benign, it is said to be an "adenoma". The term "adenoma", as used herein, thus relates to a benign epithelial neoplasm. Adenomas are usually well circumscribed, they can be flat or polypoid and the neoplastic cells do not infiltrate or invade adjacent tissue. The term "adenoma" is understood as equivalent to "non-progressed adenoma".

Benign adenomas typically do not invade other tissue and rarely metastasize. Malignant adenocarcinomas invade other tissues and often metastasize given enough time to do so. Malignant cells are often characterized by progressive and uncontrolled growth. They can spread locally or through the blood stream and lymphatic system to other parts of the body.

The term "progressed adenoma" refers to an adenoma that harbors a focus of a cancer. This is also called a "malignant polyp". Colorectal adenomas are common in the elderly population, but only a small proportion of these pre-malignant tumors (estimated approximately 5%) progresses to malignant tumors (i.e. colorectal adenocarcinoma).

The term "colorectal", as used herein, relates to the colon and/or the rectum, i.e. the complete large intestine.

In one aspect, the present invention relates to *an in vitro* method for diagnosing in a subject an adenocarcinoma associated with a chromosomal aberration on chromosome 20q, the method comprising the steps of: (a) detecting in a test sample obtained from the subject the expression level(s) of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397); and (b) comparing the expression level(s) obtained in step (a) to a control sample, wherein an elevated expression level of any one of the marker genes in the test sample as compared to the control level is indicative of an adenocarcinoma associated with a chromosomal aberration on chromosome 20q in the subject.

The term "marker gene", as used herein, is a gene whose expression level is modified, preferably elevated, in an adenocarcinoma associated with a chromosomal aberration on chromosome 20q in comparison to a control level or state.

The term "control level" (or "control state"), as used herein, relates to an expression level which may be determined at the same time as the test sample by using
(a) sample(s) previously collected and stored from a subject/subjects whose disease state, e.g. non-cancerous, is/are known.

The term "non-cancerous", as used herein, relates in the context of the present invention to a condition in which neither benign nor malign proliferation can be detected. Suitable means for said detection are known in the art. Preferably, the term "non-cancerous" excludes a benign proliferation state as present in adenomas.

Alternatively, the control level may be determined by a statistical method based on the results obtained by analyzing previously determined expression level(s) of the marker genes of the present invention in samples from subjects whose disease state is known. Furthermore, the control level can be derived from a database of expression patterns from previously tested subjects or cells. Moreover, the expression level of the marker genes of the present invention in a biological sample to be tested may be compared to multiple control levels, whose control levels are determined from multiple reference samples. It is preferred to use a control level determined from a reference sample derived from a tissue type similar to that of the patient-derived biological sample. It is particularly preferred to use sample(s) derived from a subject/subjects whose disease state is non-cancerous or derived from a subject/subjects whose disease state is non-cancerous as defined herein above. In another embodiment of the present invention, the control level can be determined from a reference sample derived from a subject who has been diagnosed to suffer from adenoma.

Moreover, it is preferred, to use the standard value of the expression levels of any of the marker genes of the present invention in a population with a known disease state. The standard value may be obtained by any method known in the art. For example, a range of mean ± 2 SD (standard deviation) or mean ± 3 SD may be used as standard value.

Furthermore, the control level may also be determined at the same time with the test sample by using (a) sample(s) previously collected and stored from a subject/subjects whose disease state is/are known to be cancerous, in particular who have independently been diagnosed to suffer from an adenocarcinoma or an adenocarcinoma associated with a chromosomal aberration on chromosome 20q.

Furthermore, the control level may also be determined by using (a) sample(s) previously collected and stored from a subject/subjects who are known to have chromosomal aberrations, preferably gains, on chromosome 20q. Means and methods for the detection of a chromosomal aberration on chromosome 20q independently of the expression level of the marker genes of the present invention are described herein below.

In the context of the present invention, a control level determined from a biological sample that is known not to be cancerous is called "normal control level". If the control level is determined from a cancerous biological sample, i.e. a sample from a subject for which adenocarcinoma associated with a chromosomal aberration on chromosome 20q was diagnosed independently, it may be designated as "cancerous control level".

When the expression level of any one the maker genes of the present invention is increased compared to the normal control level as defined herein above or is similar to the cancerous control level as defined herein above, the subject may be diagnosed to be suffering from developing an adenocarcinoma associated with a chromosomal aberration on chromosome 20q. In a further embodiment, an additional similarity in the overall gene expression pattern between the sample and the reference, which is cancerous, indicates that the subj ect is suffering from an adenocarcinoma associated with a chromosomal aberration on chromosome 20q.

The difference between the expression levels of a test biological sample and the control level can be normalized to the expression level of further control nucleic acids, e.g. housekeeping genes whose expression levels are known not to differ depending on the cancerous or non-cancerous state of the cell. Exemplary control genes include *inter alia* β-actin, glycerinaldehyde 3-phosphate dehydrogenase, and ribosomal protein P1.

The term "elevated expression level" in the context of the present invention denotes an increase of the expression level. Expression levels are deemed to be "elevated" when the gene expression increases by, for example, 5%, 6%, 7%, 8%, 9%, 10%, 15%, 20%, 25%, 30%, 40%, 50%, or more than 50% from a control level, or at least 0.1 fold, at least 0.2 fold, at least 1 fold, at least 2 fold, at least 5 fold, or at least 10 fold or more in comparison to a control level.

In the context of the present invention, the term "diagnosing" is intended to encompass predictions and likelihood analysis. The present method is intended to be used clinically in making decisions concerning treatment modalities, including therapeutic intervention, diagnostic criteria such as disease stages, and disease monitoring and surveillance for the disease. According to the present invention, an intermediate result for examining the condition of a subject may be provided. Such intermediate result may be combined with additional information to assist a doctor, nurse, or other practitioner to diagnose that a subject suffers from the disease. Alternatively, the present invention may be used to detect cancerous cells in a subject-derived tissue, and provide a doctor with useful information to diagnose that the subject suffers from the disease.

A subj ect to be diagnosed by the present method is a mammal, preferably a human being.

Biological sample may be collected or obtained from the subject to be diagnosed to perform the diagnosis. Any biological material can be used as the biological sample for the determination so long as it includes the objective transcription or translation product of the marker genes of the present invention. The biological samples may include body tissues and fluids, such as blood, sputum, and urine. Furthermore, the biological sample may contain a cell extract derived from or a cell population including an epithelial cell, preferably a cancerous epithelial cell or an epithelial cell derived from tissue suspected to be cancerous. Even more preferably the biological sample contains a cell population derived from a glandular tissue. Furthermore, the cell may be purified from the obtained body tissues and fluids if necessary, and then used as the biological sample. According to the present invention, the expression level of the marker genes of the present invention is determined in the subject-derived biological sample(s).

The sample used for detection in the *in vitro* methods of the present invention should generally be collected in a clinically acceptable manner, preferably in a way that nucleic acids (in particular RNA) or proteins are preserved. The samples to be analyzed are typically colorectal biopsies or resections. Intact cells or a cell lysate from tumor tissue may also detach from the colon without intervention and will end up in the faeces. Accordingly, stool samples are also considered as a suitable source for isolating RNA. Furthermore, colorectal adenocarcinoma cells may migrate into other tissues. Consequently, also blood and other types of sample can be used. A biopsy or resection may contain a majority of adenoma cells and only a minority of adenocarcinoma cells. To increase the signal/background ratio, a resection can be divided into different sub-samples prior to analysis. Even if the total number of carcinoma cells in the biopsy or resection is limited, at least one of the sub-samples may contain an increased ratio of adenocarcinoma versus adenoma cells. Samples, in particular after initial processing may be pooled. However, also non-pooled samples may be used.

In a specific embodiment of the invention, adenomatous polyp biopsies or resections are obtained. For *in vitro* protein expression analysis, cells or cell lysates of biopsies or resections may be used. Accordingly, the localization of the protein in the cell or the function of the protein to be assayed is of no importance for the analysis. The presence of adenocarcinoma cells in a patient is typically reflected by the presence of elevated or decreased levels of certain proteins secreted by adenocarcinoma cells. Such proteins can be present in blood, urine, sweat and other parts of the body. Equally, adenocarcinoma cells will release proteins to the colon lumen. In addition, intact adenocarcinoma cells or their lysed content may be released to the intestinal tract, and will be present in the faeces which can be used as a source for *in vitro* protein analysis. However, contrary to nucleic acids, proteins cannot be amplified. Accordingly, it is envisaged that, in particular embodiments, the methods of the invention comprise an enrichment step, more particularly an enrichment of adenocarcinoma material. For instance, a sample can be contacted with ligands specific for the cell membrane or organelles of adenoma and adenocarcinoma cells, functionalized for example with magnetic particles. The material concentrated by the magnetic particles can then be analyzed for the detection of marker proteins.

The term "at least one of the marker genes" relates in one embodiment to the expression level of the entire group of marker genes, i.e. an averaged expression level, preferably normalized to a suitable control as defined herein above. The term may also relate to any subgroup of the marker genes, e.g., *RNPC1* and *TCFL5* and *C20orf24* and *AURKA*/*STK6* and *C20orf20* and *ADRH1*; or *RNPC1* and *TCFL5* and *C20orf24* and *AURKA*/*STK6* and *C20orf20;* or *RNPC1* and *TCFL5* and *C20orf24* and *AURKA*/*STK6*; or *RNPC1* and *TCFL5* and *C20orf24*; or *RNPC1* and *TCFL5*; or *RNPC1* and *C20orf24* and *AURKA*/*STK6* and *C20orf20* and *ADRH1* and *TH1L;* or *RNPC1* and *C20orf24* and *AURKA*/*STK6* and *C20orf20* and *ADRH1*; or *RNPC1* and *C20orf24* and *AURKA*/*STK6* and *C20orf20*; or *RNPC1* and *C20orf24* and *AURKA*/*STK6*; or *RNPC1* and *C20orf24*; or *RNPC1* and *TCFL5* and *C20orf24* and *C20orf20* and *ADRM1* and *TH1L;* or *RNPC1* and *TCFL5* and *C20orf24* and *C20orf20* and *ADRH1*; or *RNPC1* and *TCFL5* and *C20orf24* and *C20orf20*; or *RNPC1* and *TCFL5* and *C20orf24* and *AURKA*/*STK6* and *ADRH1* and *TH1L;* or *RNPC1* and *TCFL5* and *C20orf24* and *AURKA*/*STK6* and *ADRH1*; or *RNPC1* and *TCFL5* and *C20orf24* and *C20orf20* and *TH1L;* or *TCFL5* and *C20orf24* and *AURKA*/*STK6* and *C20orf20* and *ADRH1* and *TH1L,* or *TCFL5* and *C20orf24* and *AURKA*/*STK6* and *C20orf20* and *ADRM1*; or *TCFL5* and *C20orf24* and *AURKA*/*STK6* and *C20orf20*; or *TCFL5* and *C20orf24* and *AURKA*/*STK6*, or *TCFL5* and *C20orf24*; or *TCFL5* and *AURKA*/*STK6* and *C20orf20* and *ADRH1* and *TH1L;* or *TCFL5* and *AURKA*/*STK6* and *C20orf20* and *ADRH1*; or *TCFL5* and *AURKA*/*STK6* and *C20orf20*; or *TCFL5* and *AURKA*/*STK6*; or *TCFL5* and *C20orf24* and *C20orf20* and *ADRM1* and *TH1L;* or *TCFL5* and *C20orf24* and *C20orf20* and *ADRM1*; or *TCFL5* and *C20orf24* and *C20orf20*; or *TCFL5* and *C20orf24* and *AURKA*/*STK6* and *ADRH1* and *TH1L;* or *TCFL5* and *C20orf24* and *AURKA*/*STK6* and *ADRM1*; or *TCFL5* and *C20orf24* and *AURKA*/*STK6* and *C20orf20* and *TH1L;* or *C20orf24* and *AURKA*/*STK6* and *C20orf20* and *ADRH1* and *TH1L;* or *C20orf24* and *AURKA*/*STK6* and *C20orf20* and *ADRH1*; or *C20orf24* and *AURKA*/*STK6* and *C20orf20*; or *C20orf24* and *AURKA*/*STK6*; or *C20orf24* and *C20orf20* and *ADRH1* and *TH1L;* or *C20orf24* and *C20orf20* and *ADRM1*; or *C20orf24* and *C20orf20*; or *C20orf24* and *AURKA*/*STK6* and *ADRH1* and *TH1L*; or *C20orf24* and *AURKA*/*STK6* and *ADRH1*; or *AURKA*/*STK6* and *C20orf20* and *ADRH1* and *TH1L*; or *A URKAlSTK6* and *ADRH1* and *TH1L*; or *AURKA*/*STK6* and *ADRH1*; or *AURKA*/*STK6* and *C20orf20* and *TH1L*; or *AURKA*/*STK6* and *C20orf20* and *ADRH1*; or *AURKA*/*STK6* and *C20orf20*; or *C20orf20 and ADRM1* and *TH1L;*or *C20orf20* and *TH1L;* or *C20orf20* and *ADRH1*; or *ADRM1* and *TH1L;* or *RNPC1* and *AURKA*/*STK6;* or *RNPC1* and *C20orf20*, or *RNPC1* and *ADRM1*; or *TCFL5* and *C20orf20*; or *TCFL5* and *ADRM1*; or *C20orf24* and *ADRM1* etc., or any individual marker gene. Particularly preferred is the subgroup *RNPC1* and *TCFL5* or any other combination of the marker genes of the present invention which comprises as elements *RNPC1* and *TCFL5*. Such a combination may comprise in addition to *RNPC1* and *TCFL5* also *C20orf24* and/or *AURKA*/*STK6* and/or *C20orf20* and/or *ADRM1* and/or *TH1L*.

In case a subgroup is to be employed, e.g. one of the above mentioned, the expression level is to be seen as the expression level of the entire subgroup of marker genes, i.e. an averaged expression level, preferably normalized to a suitable control as defined herein above.

Surprisingly it has been found that a combination of at least two of the above mentioned markers allow correctly distinguishing adenomas from adenocarcinomas in at least 85%, preferably 88% of the cases examined according to the method of the present invention. This preferably relates to a combination that comprises at least *RNPC1* and *TCFL5*.

In a particularly preferred embodiment of the present invention, the expression level(s) of at least marker genes *RNPC1* (Genbank accession # NM_017495) and *TCFL5* (Genbank accession # NM_006602) are detected, wherein elevated expression levels of both said marker genes in the test sample, compared to the control level are indicative for adenocarcinoma associated with a chromosomal aberration on chromosome 20q or variation of this indication as described herein below, i.e. a predisposition to adenocarcinoma associated with a chromosomal aberration on chromosome 20q, a progression of adenoma to adenocarcinoma associated with a chromosomal aberration on chromosome 20q or a predisposition for a progression of adenoma to adenocarcinoma associated with a chromosomal aberration on chromosome 20q. The expression level may preferably be averaged over the expression level of both marker genes and/or normalized with an appropriate control as described herein above and herein below.

The term "chromosomal aberration", as used in the context of the present invention, relates to a chromosomal rearrangement resulting in a loss or gain of chromosomal portions or regions, i.e. a deletion or duplication of regions in the chromosome. A deletion or loss may be a deletion of chromosomal regions of a size between about 0.3 kb and several Mb, e.g. between 0.3 kb and 50 Mb, or any sub-range thereof, e.g., 0.3 kb - 40 Mb, 0.3 kb - 30 Mb, 0.3 kb - 20 Mb, 0.3 kb - 15 Mb, 0.3 kb - 10Mb, 0.3 kb - 5 Mb, 0.3 kb - 2 Mb or 0.3kb -1 Mb.

The term "adenocarcinoma associated with a chromosomal aberration on chromosome 20q" relates to a link or relationship between the presence of adenocarcinoma or any disease state(s) thereof, as defined herein above, and a chromosomal rearrangement on chromosome 20q. Thus, if an adenocarcinoma is detected according to means and method of the present invention, the presence of the disease is linked to a chromosomal aberration on chromosome 20, in particular in the region 20q. In a preferred embodiment the term relates to a link or relationship between the presence of adenocarcinoma or any disease state(s) thereof, as defined herein above, and a chromosomal rearrangement on 20q11.22 - 20q11.23 and/or at position 20q13.31 - 20q13.33. The chromosomal rearrangement or aberration may be a gain or loss, a 1 or several fold duplication or deletion, preferably a chromosomal gain.

The sequences of the marker genes or marker loci of the present invention, i.e. *RNPC1, TCFL5, C20orf24, AURKA*/*STK6, C20orf20, ADRM1*, and *TH1L* are known from the literature and have, for example, been deposited in gene databases such as Genbank under the accession numbers (#) NM_017495, NM_006602, NM_018840, NM_003600, NM_018270, NM_007002 and NM_016397, respectively. The genes or loci may also be designated by synonyms, which are known to the person skilled in the art and can be derived, for example, from the above mentioned database entries. These synonyms are also meant when reference is made to the indicated marker genes. These synonyms are also encompassed by the embodiments of the present invention.

All of these marker genes or marker loci map to chromosome 20, in particular to chromosome 20q and accordingly establish an association between adenocarcinoma and a chromosomal aberration on chromosome 20q as has been shown *in extenso* in the examples of the present invention.

The present invention refers in a preferred embodiment to the diagnosis of specific adenocarcinoma-associated disease states, i.e. disease states that are (closely) related but not identical to adenocarcinoma. The term "adenocarcinoma-associated disease states", as used herein, thus relates particularly to a predisposition for developing an adenocarcinoma associated with a chromosomal aberration on chromosome 20q, a progression of an adenoma to an adenocarcinoma associated with a chromosomal aberration on chromosome 20q or a predisposition for a progression of an adenoma to an adenocarcinoma associated with a chromosomal aberration on chromosome 20q.

A "predisposition for developing an adenocarcinoma associated with a chromosomal aberration on chromosome 20q" in the context of the present invention is a state of risk of developing adenocarcinoma associated with a chromosomal aberration on chromosome 20q. Preferably a predisposition for developing an adenocarcinoma associated with a chromosomal aberration on chromosome 20q may be present in cases in which the marker gene expression level as defined herein above is below a cancerous control level as defined herein above, i.e. a reference expression level derived from tissues or samples of a subject which evidently suffers from adenocarcinoma associated with a chromosomal aberration on chromosome 20q. The term "below" in this context relates to an expression level of a marker gene which is reduced by about 40 % to 80% in comparison to such a cancerous control level, more preferably to a reduction of about 50% The reduction may be calculated over the averaged expression level or the entire group of marker genes. Alternatively, a reduction of 40 % to 80% or preferably 50% of only one marker gene or a subgroup of the marker genes, e.g. those subgroups mentioned herein above, of the present invention may also be considered as indicative for a predisposition for developing an adenocarcinoma associated with a chromosomal aberration on chromosome 20q.

The term "progression of an adenoma to an adenocarcinoma associated with a chromosomal aberration on chromosome 20q", as used herein, relates to a state in which the expression level of one or several or all of the marker genes of the present invention are modified, preferably increased, in a test sample in comparison to an adenoma control sample. Preferably, the term relates to cases in which the marker gene expression level, as defined herein above, is elevated by a value of between 3% to 50%, preferably by a value of 25% in comparison to an adenoma control sample. The increase may be calculated over the averaged expression level or the entire group of marker genes. Alternatively, an increase of 3% to 50%, preferably of 25%, of only one marker gene or a subgroup of the marker genes, e.g., those subgroups mentioned herein above, of the present invention may also be considered as indicative for a progression of an adenoma to an adenocarcinoma associated with a chromosomal aberration on chromosome 20q.

The term "predisposition for a progression of an adenoma to an adenocarcinoma associated with a chromosomal aberration on chromosome 20q", as used herein, relates to a similar state as the progression of adenoma to adenocarcinoma associated with a chromosomal aberration on chromosome 20q. However, in said condition the marker gene expression level, as defined herein above, is elevated by a value of between 1% and 15%, preferably by a value of 10% in comparison to an adenoma control sample. The increase may be calculated over the averaged expression level or the entire group of marker genes. Alternatively, an increase of 1% to 15%, preferably by a value of 10% of only one marker gene or a subgroup of the marker genes, e.g., those subgroups mentioned herein above, of the present invention may also be considered as indicative for a predisposition for a progression of an adenoma to an adenocarcinoma associated with a chromosomal aberration on chromosome 20q.

In a further preferred embodiment of the present invention the chromosomal aberration is an aberration at chromosomal position 20q11.22 - 20q11.23 and/or at position 20q13.31 - 20q13.33. These locations are known to the person skilled in the art and can be derived from any genetic map of chromosome 20.

In a further embodiment, the present invention relates to a method for diagnosing adenocarcinoma associated with a chromosomal aberration on chromosome 20q, in which the chromosomal aberration is a chromosomal gain. As has been set forth herein above, a chromosomal gain is to be seen as a duplication of chromosomal regions or portions thereof. The chromosomal gain may be a single, double or triple duplication of chromosomal regions. A "chromosomal gain" in the context of this embodiment may particularly be a duplication of (one or more) chromosomal regions of a size between about 0.3 kb and several Mb, e.g., between 0.3 kb and 50 Mb, or any sub-range thereof, e.g., 0.3 kb - 40 Mb, 0.3 kb - 30 Mb, 0.3 kb - 20 Mb, 0.3 kb - 15 Mb, 0.3 kb - 10Mb, 0.3 kb - 5 Mb, 0.3 kb - 2 Mb or 0.3kb - 1 Mb. The duplicated or gained regions may be derived from the same chromosome or from different chromosomes. Preferably, they are from the same chromosome.

Generally, the determination of the expression level of marker genes in a patient sample may be accomplished by any means known in the art. In preferred embodiment of the present invention the expression level(s) of the marker gene(s) is (are) determined by any one or more of the methods selected from the group consisting of detecting a mRNA encoded by the marker gene(s); detecting a protein encoded by the marker gene(s); and detecting a biological activity of a protein encoded by the marker gene(s). For example, expression levels of the marker genes may be assessed by separation of nucleic acid molecules (e.g. RNA or cDNA) obtained from the sample in agarose or polyacrylamide gels, followed by hybridization with marker gene specific oligonucleotide probes. Alternatively, the difference in expression level may be determined by the labeling of nucleic acid obtained from the sample followed by separation on a sequencing gel. nucleic acid samples are placed on the gel such that patient and control or standard nucleic acid are in adjacent lanes. Comparison of expression levels is accomplished visually or by means of a densitometer.

Methods for the detection of mRNA are known to the person skilled in the art or can be derived from standard textbooks, for example, Sambrook et al., Molecular Cloning: A Laboratory Manual, 2001, Cold Spring Harbor Laboratory Press. Typically, Northern blot analysis may be used for such a purpose. Preferably, mRNA may be detected in a microarray approach, e.g. sample nucleic acids derived from subjects to be tested are processed and labeled, preferably with a fluorescent label. Subsequently, such nucleic acid molecules are used in a hybridization approach with immobilized capture probes corresponding to one, more or all of the marker genes of the present invention. Suitable means for carrying out microarray analyses are known to the person skilled in the art. Typically, microarray based expression profiling may be carried out, for example, by the method as disclosed in "Microarray Biochip Technology" (Schena M., Eaton Publishing, 2000). A DNA array comprises immobilized high-density probes to detect a number of genes. The probes on the array are complementary to one or more parts of the sequence of a marker gene, or to the entire coding region of the marker gene. In the present invention, any type of polynucleotide can be used as probes for the DNA array. Typically, cDNAs, PCR products, and oligonucleotides are useful as probes. Thus, expression levels of a plurality of genes can be estimated at the same time by a single-round analysis.

A DNA array-based detection method generally comprises the following steps: (1) Isolating mRNA from a sample and optionally converting the mRNA to cDNA, and subsequently labeling this RNA or cDNA. Methods for isolating RNA, converting it into cDNA and for labeling nucleic acids are described in manuals for micro array technology. (2) Hybridizing the nucleic acids from step 1 with probes for the marker genes. The nucleic acids from a sample can be labeled with a dye, such as the fluorescent dyes Cy3 (red) or Cy5 (blue). Generally a control sample is labeled with a different dye. (3) Detecting the hybridization of the nucleic acids from the sample with the probes and determining at least qualitatively, and more particularly quantitatively, the amounts of mRNA in the sample for the different marker genes investigated. The difference in the expression level between sample and control can be estimated based on a difference in the signal intensity. These can be measured and analyzed by appropriate software such as, but not limited to the software provided for example by Affymetrix.

There is no limitation on the number of probes corresponding to the marker genes used, which are spotted on a DNA array. Also, a marker gene can be represented by two or more probes, the probes hybridizing to different parts of a gene. Probes are designed for each selected marker gene. Such a probe is typically an oligonucleotide comprising 5-50 nucleotide residues. Longer DNAs can be synthesized by PCR or chemically. Methods for synthesizing such oligonucleotides and applying them on a substrate are well known in the field of micro-arrays. Genes other than the marker genes may be also spotted on the DNA array. For example, a probe for a gene whose expression level is not significantly altered may be spotted on the DNA array to normalize assay results or to compare assay results of multiple arrays or different assays.

The detection of proteins encoded by the marker gene or genes may be carried out via antibody detection techniques known in the art. For the analysis at the protein level, every marker gene described in the present invention can in principle be used, although some proteins may be less suitable, because of factors such as limited solubility, very high or small molecular weight or extreme iso-electric point. Determination of expression level of a marker gene at the protein level can be accomplished, for example, by the separation of proteins from a sample on a polyacrylamide gel, followed by identification of a specific marker gene-derived protein using appropriate antibodies in a Western blot analysis. Alternatively, proteins can be separated by two-dimensional gel electrophoresis systems. Two-dimensional gel electrophoresis is well known in the art and typically involves iso-electric focusing along a first dimension followed by SDS-PAGE electrophoresis along a second dimension. The analysis of 2D SDS-PAGE gels can be performed by determining the intensity of protein spots on the gel, or can be performed using immune detection. In other embodiments, protein samples are analyzed by mass spectroscopy.

Alternatively, antibodies directed against the proteins encoded by any one of the marker genes of the present invention may be generated. Preferably, monoclonal antibodies are obtained. Subsequently, such specifically binding antibodies may be used to detect the proteins encoded by the marker genes. In a specific embodiment the antibodies may be stained with a dye or be labeled. Alternatively, antibodies binding proteins encoded by the marker genes may also be placed on a support and be immobilized. Proteins derived from samples or tissues to be analyzed may subsequently be mixed with the antibodies. A detection reaction may then be carried out, e.g. with a second specific antibody.

In addition, ligands to the proteins encoded by the marker genes of the present invention may be used for a detection of said proteins. Such ligands may preferably be labeled in order to allow the detection of a protein-ligand interaction.

The detection of a biological activity of a protein encoded by the marker genes of the present invention may be carried out by employing molecular or enyzmatic assays specific to the corresponding functions of the marker genes. These functions may be derived from the Genbank database entries mentioned in the context of the marker genes of the present invention or from corresponding literature, e.g. the citations mentioned herein below. For instance, *TCFL5* is a transcription factor (Siep, M. et al. (2004) Nucleic Acids Res. 32, 6425-6436), *C20orf20* is a factor being involved in transcriptional regulation (Cai, Y. et al. (2003) J. Biol. Chem. 278, 42733-42736). *TH1L* product is involved in regulation of A-Raf kinase (Liu, W. et al. (2004) J. Biol. Chem. 279, 10167-10175). *ADRM1* encodes for a putative cell adhesion molecule that recently was shown to be component of the 26S proteosome (Jorgensen, J.P. et al. (2006) J. Mol. Biol. 360, 1043-1052). *RNPC1* product is predicted to bind to RNA, based on sequence motifs and *C20orf24* interacts with Rab-5. *AURKA* has been well characterized and is involved in cell cycle regulation. It has been shown to be amplified in CRC (Bischoff, J.R. et al. (1998) EMBO J. 17, 3052-3065) and its over-expression induces centrosome amplification, aneuploidy and transformation *in vitro* (Zhou, H. et al. (1998) Nat. Genet. 20, 189-193). Moreover, inhibiting *AURKA* by RNA interference lead to growth suppression of human pancreatic cancer cells (Hata, T. et al. (2005) Cancer Res. 65, 2899-2905). Knocking down *TCFL5* resulted in suppression of the number of multicellular HT29 tumour spheroids, supporting its role in cancer development (Dardousis, K. et al. (2007) Mol. Ther. 15, 94-102). A person skilled in the art could envisage suitable and appropriate assays in order to test for the corresponding functions. For example, such assays may comprise kinase assays (e.g., for the detection of the biological function of *AURKA*/*STK6*) or transcription or transcription regulation assays (e.g., for the detection of the biological function of *TCFL5*) or RNA interaction assays (e.g., for the detection of the biological function of *RNPC1*).

The method of diagnosis of the present invention may further be combined with detection procedures for chromosomal aberrations, in particular chromosomal aberrations on chromosome 20q. Preferably, such detection procedures may be used for the detection of chromosomal aberrations at position 20q11.22 - 20q11.23 and/or at position 20q13.31 - 20q13.33. Even more preferably, such detection procedures may be used for the detection of chromosomal aberrations at the loci of the marker genes of the present invention, i.e., one or more or all of *RNPC1, TCFL5, C20orf24, AURKA*/*STK6, C20orf20, ADRM1*, and *TH1L*, which are derivable from Genbank under the accession numbers NM_017495, NM_006602, NM_018840, NM_003600, NM_018270, NM_007002, and NM_016397, respectively. The exact genetic and molecular position of these marker genes within chromosome 20q can be derived from a genomic map when searching with the indicated Genbank accession numbers. Such an approach also allows the identification of appropriate primer sequences and hybridization probes.

The term "marker gene" relates particularly to the marker gene or group of marker genes or subgroup of marker genes or individual marker gene as defined herein above. It more preferably relates to a combination that comprises at least *RNPC1* and *TCFL5*.

Further preferred details and embodiments of such a chromosomal detection are described herein below in the context of a method for diagnosing in a subject an adenocarcinoma comprising the detection of a chromosomal aberration. The therein described applicable to this method as well.

Chromosomal aberration detection procedures encompassed by the present invention comprise, for example, comparative genomic hybridization (CGH), PCR detections, multiplex ligation-dependent probe amplification (MPLA) or a loss of heterocygosity (LOH) analysis.

For instance, in a CGH procedure, genomic DNA of a test sample may be hybridized with an array of genomic clones representing the human genome. CGH is an established method, exemplified *inter alia* in the Examples section of the present application. CGH is based on the hybridization of sample DNA with DNA on a matrix. The presence of genomic aberrations is detected based on a difference in the hybridization pattern compared to a control DNA. In order to have a reliable result, nonspecific hybridization is to be avoided. This is performed e.g., by removing non-specifically bound DNA using elevated temperatures, high salt concentrations and chaotropic agents such as formamide. The values for each of these parameters depend on the degree of sequence similarity and length of the hybridizing partners. Suitable values are found in instructions of the manufacturers of CGH arrays and in reference books such as Sambrook et al., Molecular Cloning: A Laboratory Manual, 2001, Cold Spring Harbor Laboratory Press. Typical solutions contain about 50% formamide, 2x SSC, pH 7 or 0.1 M sodium phosphate, 0.1% Nonidet P40, pH 8 with SSC concentrations ranging from 0.2 to 0.01 x SSC.

In a multiplex ligation-dependent probe amplification (MPLA) approach typically two probes are used which hybridize adjacent to each other on a sample DNA. Subsequently, the probes are ligated and the ligated probes, instead of the sample, are amplified by PCR. In a particular embodiment, the probes may be selected such that target sequences of the adjacent probes are sequences within the region of chromosomal aberration. The amount of amplified product reflects the relative copy number of the target sequence. Alternatively, probes can be selected such that the size or the presence/absence of the amplicon is indicative of chromosomal aberrations. MLPA allows the use of different probe pairs, hybridizing to different parts of a chromosome (each generating an amplicon of a specific length) at the same time. Accordingly, different SROs can be detected simultaneously (Schouten, B. et al. (2002) Nucl. Acids Res. 30, e57).

Furthermore, the identification of well-defined genomic regions of interest allows a further refinement of the CGH technique whereby only probes directed to the specific region of interest are used. Accordingly, in a further aspect, the present invention provides pairs of primers which detect the duplication or loss of one or more portions or loci of chromosome 20q, in particular the loci of the marker genes of the present invention. More particularly, the present invention provides chromosome 20q or marker gene-specific primer pairs, preferably primer pairs for the loci of NM_017495, NM_006602, NM_018840, NM_003600, NM_018270, NM_007002 and NM_016397.

Chromosomal deletions may be qualitatively detected, e.g., by a forward primer located 5' and a reverse primer located 3' of a locus on chromosome 20q, preferably position 20q11.22 - 20q11.23 and/or position 20q13.31 - 20q13.33 and more preferably the loci NM_017495, NM_006602, NM_018840, NM_003600, NM_018270, NM_007002 and NM_016397.

When a deletion (or chromosomal loss) at such a locus occurs, a part of the genomic DNA between the primers is absent and results in the generation of a PCR product which is considerably smaller than in wild-type (no chromosomal loss). The PCR fragments amplified from regions with a deletion are smaller than the fragments of an intact chromosome. Such smaller fragments will be preferentially amplified, allowing a very sensitive detection. Additionally or alternatively, the elongation time in a PCR reaction can be shortened to discourage the amplification of longer PCR products.

The occurrence of a duplication, or a chromosomal gain may be detected, e.g., by a forward primer in the 3' region and a reverse primer in the 5' region of a locus on chromosome 20q, preferably position 20q11.22 - 20q11.23 and/or position 20q13.31 - 20q13.33 and more preferably the loci NM_017495, NM_006602, NM_018840, NM_003600, NM_018270, NM_007002, and NM_016397. Since these primers "point away" from each other, there will be no PCR product at all on a chromosome without duplication.

Stringency conditions for use with PCR primers may be determined by calculation of the length, GC composition and degree of sequence identity between primer and template. Based upon the predicted melting temperature of a primer, the conditions of PCR amplification are adapted. The stringency parameters in a PCR reaction are largely determined by the choice of the annealing temperature in a PCR cycle. Different software programs are available to select in a given DNA sequence a pair of PCR primers with desired melting temperature, which are specific and which do not hybridise with each other, or form hairpins. Optionally, the specificity of a PCR reaction in increased by performing so-called nested PCR. Kits for amplification of genomic DNA are available from, for example, Roche or Stratagene.

According to another embodiment, the methods of the present invention comprise detecting the loci on chromosome 20q as described herein above by quantitative PCR. Using primers annealing to a sequence located within a locus of interest on chromosome 20q, the quantitative expression of this sequence in a sample can be compared to a control (same region in a control sample or other region). Similarly, using MLPA, primer pairs can be used which target a sequence within a region of chromosomal loss or gain (MLPA), resulting in the generation of a relative amount of amplicon which reflects the relative copy number of the target sequence.

A specific target sequence located within the loci on chromosome 20q as described herein above can be determined by the skilled person. While in essence any part of genomic DNA is suitable as a target for amplification, in particular embodiments, a part of a gene, more particularly at least a part of at least one exon is used as target for amplification.

In a further aspect the present invention relates to *an in vitro* method for diagnosing in a subject an adenocarcinoma, the method comprising: (a) detecting in a test sample obtained from the subject a chromosomal aberration, preferably a gain, on chromosome 20q; and further comprising - preferably in case a chromosomal aberration, preferably gain, is detected on chromosome 20, more preferably in case a chromosomal aberration or gain is detected on chromosome 20q, even more preferably in case a chromosomal aberration or gain is detected at position 20q11.22-20q11.23 and/or at position 20q13.31-20q13.33 - the steps of (b) detecting in said sample the expression level(s) of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397); and (c) comparing the expression level(s) obtained in step (b) to a control level, wherein an elevated expression level of any one of the marker genes in the test sample as compared to the control level is indicative of an adenocarcinoma.

Such a method may encompass any steps or procedures mentioned herein above with regard to the detection of chromosomal aberrations or the detection of the expression level(s) of the marker genes. The term "marker gene" relates particularly to the marker gene or group of marker genes or subgroup of marker genes or individual marker gene as defined herein above. It more preferably relates to a combination that comprises at least *RNPC1* and *TCFL5*. A combination of at least two of the above mentioned markers, preferably *RNPC1* and *TCFL5*, allow correctly distinguishing adenomas from adenocarcinomas in at least 85%, preferably 88%, more preferably 90% and even more preferably 95% of the cases examined according to this aspect of the present invention.

In a preferred embodiment of this method the execution of the step of detecting in the examined sample the expression level(s) of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397); and the subsequent comparison of the expression level(s) obtained to a control level may be made dependent on the outcome of the detection of chromosomal aberrations on chromosome 20, preferably 20q or the positions 20q11.22-20q11.23 and/or 20q13.31-20q13.33, i.e. a medical practitioner or any person working with such a diagnosing method may decide upon receiving results from a chromosomal aberration test as defined herein above, to continue with a testing of the expression level(s) of any one of the marker genes of the present invention. Such a decision may depend on the size of the chromosomal aberration, its bounderies or the loci involved. Preferably, a testing of the expression levels is carried out if at least between about 0.5% to about 100% of chromosome 20 is aberrated, more preferably, if about 0.5% to about 100% of chromosome 20q is aberrated, even more preferably, if between about 50% and 100% of chromosome 20q is duplicated. In a particularly preferred embodiment the detection of expression levels of any one of the marker genes as defined herein above may be carried out if at least chromosomal regions 20q11.22-20q11.23 and/or 20q13.31-20q13.33 are at a level of about 5% to 100% duplicated, e.g. of about 90%, of about 80%, of about 70%, of about 60%, of about 50%, of about 40%, of about 30%, of about 20% or of about 10%.

In another preferred embodiment, the present invention relates to an *in vitro* method for diagnosing in a subject an adenocarcinoma comprising the detection of a chromosomal gain on chromosome 20q as described above, wherein the detection of said chromosomal gain on chromosome 20q is performed by comparative genomic hybridization (CGH), PCR detection or multiplex ligation-dependent probe amplification (MPLA). CGH, PCR detection and MPLA techniques have already been described herein above.

In a further preferred embodiment, the present invention relates to a kit for diagnosing adenocarcinoma comprising means for detecting the expression of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397).

Typically, the kits of the present invention contain one or more agents allowing the specific detection of the marker genes as defined in the claims. The nature of the agents is determined by the method of detection for which the kit is intended. Where detection at the DNA/RNA method is intended, the agents are typically marker-specific primers or probes, which may be optionally labeled according to methods known in the art (e.g., with a fluorescent label, a luminescent label, an enzyme label etc.). Where detection is at the protein level, agents are typically antibodies or compounds containing an antigen-binding fragment of an antibody. However protein expression can also be detected using other compounds that specifically interact with the marker of interest, such as specific substrates (in case of enzymes) or ligands (for receptors). Preferably, a kit of the present invention comprises detection reagents for at least of the marker genes as mentioned above. Such detection reagents comprise, for example, buffer solutions, labels or washing liquids etc. Furthermore, the kit may comprise an amount of a known nucleic acid molecule, which can be used for a calibration of the kit. Additionally, the kit may comprise an instruction leaflet.

In another preferred embodiment, the kit may additionally or alternatively comprise means for the detection of chromosomal aberrations as described herein above. Typically, such a kit may comprise PCR reagents and/or fluorescent and/or radioactive labels as well as appropriate buffer solutions. Such ingredients are known to the person skilled in the art and may vary depending on the detection method carried out.

According to a further embodiment of the present invention, an agent for treating or preventing adenocarcinoma may be identified by a method comprising the steps of contacting a test agent with one or more cells expressing any one or more of the marker genes *RNPC1* (Genbank accesion # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397); detecting the expression level(s) of the one or more marker genes; and selecting a test agent that reduces the expression level(s) of any one or more of the marker gene as compared to that (those) detected in the absence of the test agent. The test cell may be any suitable cell, e.g. an epithelial cell. A decrease in the expression level of the marker gene or the activity of its gene product as compared to a control level in the absence of the test compound indicates that the test compound may be used to reduce symptoms of cancer, preferably of adenocarcinoma.

An agent identified by the screening method of the present invention is an agent that is expected to inhibit the expression of one, more of all of the marker genes of the present invention or the activity of the translation product of these genes, and thus, is a candidate for treating or preventing diseases attributed to, for example, cell proliferative diseases, such as cancer. The agents are in particular expected to treat and/or prevent an adenocarcinoma. Namely, the agents identified through the present methods are expected to have a clinical benefit and can be further tested for an ability to prevent cancer cell growth in animal models or test subjects. In the context of the present invention, agents to be identified through the present screening methods may be any compound or composition, including several compounds. Furthermore, the test agent exposed to a cell or protein according to the screening methods of the present invention may be a single compound or a combination of compounds. When a combination of compounds is used in the methods, the compounds may be contacted sequentially or simultaneously.

Any test agent, for example, cell extracts, cell culture supernatant, products of fermenting microorganism, extracts from marine organism, plant extracts, purified or crude proteins, peptides, non-peptide compounds, synthetic compounds (including nucleic acid constructs, such as antisense RNA, siRNA, ribozymes, etc.) and natural compounds can be used in the screening methods of the present invention. The test agent of the present invention can be also obtained using any of the numerous approaches in combinatorial library methods known in the art, including, but not limited to, (1) biological libraries, (2) spatially addressable parallel solid phase or solution phase libraries, (3) synthetic library methods requiring deconvolution, (4) the "one-bead one-compound" library method and (5) synthetic library methods using affinity chromatography selection. The biological library methods using affinity chromatography selection is limited to peptide libraries, while the other four approaches are applicable to peptide, non-peptide oligomer or small molecule libraries of compounds (Lam, L. (1997) Anticancer Drug Des 12, 145-167). Examples of methods for the synthesis of molecular libraries can be found in the art (DeWitt, K. et al. (1993) Proc. Natl. Acad. Sci. USA 90, 6909-6913; Erb, B. et al. (1994) Proc. Natl. Acad. Sci. USA 91, 11422-11426; Zuckermann, G. et al, (1994) J. Med. Chem. 37, 2678-2685). Libraries of compounds may be presented in solution (Houghten, K. (1992) Bio/Techniques 13, 412-421) or on beads (Lam, L. (1991) Nature 354, 82-84), chips (Fodor, S. (1993) Nature 364, 555-556), bacteria, spores, plasmids (Cull, C. et al. (1992) Proc. Natl. Acad. Sci. USA 89, 1865-1869) or phages (Scott, C. and Smith, S. (1990) Science 249, 386-390; Devlin, F. (1990) Science 249, 404-406).

A compound in which a part of the structure of the compound identified by any of the present screening methods is converted by addition, deletion and/or replacement, is included in the agents obtained by the screening methods of the present invention.

Furthermore, when the screened test agent is a protein, or a DNA encoding a protein, either the whole amino acid sequence of the protein may be determined to deduce the nucleic acid sequence coding for the protein, or partial amino acid sequence of the obtained protein may be analyzed to prepare an DNA oligonucleotide as a probe based on the sequence, and screen cDNA libraries with the probe to obtain a DNA encoding the protein. The obtained DNA may then be used in preparing the test agent which is a candidate for treating or preventing cancer, particularly adenocarcinoma.

According to the finding of the present inventors, the expression of the marker genes described herein above is typical for the growth of adenocarcinoma cells. Therefore, it was considered that agents which suppress the function of the polypeptide encoded by the gene may inhibit the growth and/or survival of such cancer cells, and find use in treating and/or preventing adenocarcinoma or related cancer types Thus, the present invention provides methods of identifying an agent for treating or preventing adenocarcinoma, using the proteins encoded by the marker genes of the present invention. In addition to these proteins, also protein fragments may be used in the context of the present screening methods, so long as at least one biological activity of natural occurring marker gene-derived proteins is retained to at least 80%, preferably at least 90%, and particularly at least 95% as compared to the full-length counterpart..

The polypeptide or fragments thereof may be further linked to other substances so long as the resulting polypeptide and fragments retain at least one biological activity of the originating peptide. Usable substances include: peptides, lipids, sugar and sugar chains, acetyl groups, natural and synthetic polymers, etc. These kinds of modifications may be performed to confer additional functions or to stabilize the polypeptide and fragments.

The polypeptide or fragments used for the present method may be obtained from nature as naturally occurring proteins via conventional purification methods or through chemical synthesis based on the selected amino acid sequence. Alternatively, the protein may be obtained by the adoption of any known genetic engineering methods for producing polypeptides. For example, first, a suitable vector including a polynucleotide encoding the objective protein in an expressible form (e.g., downstream of a regulatory sequence including a promoter) may be prepared, transformed into a suitable host cell, and then the host cell may be cultured to produce the protein. More specifically, a gene encoding a marker gene-derived protein is expressed in host (e.g., an animal) cells by inserting the gene into a vector for expressing foreign genes, such as pSV2neo, pcDNA1, pcDNA3.1 , pCAGGS, or pCD8. A promoter may be used for the expression. Any commonly used promoters may be employed including, for example, the SV40 early promoter, or the CAG promoter. The introduction of the vector into host cells to express the marker gene can be performed according to any methods, for example, the electroporation method, the calcium phosphate method or the DEAE dextran method. A correspondingly produced polypeptide may be contacted with a test agent as described herein above.

An agent that binds to a protein is likely to alter the expression of the gene coding for the protein or the biological activity of the protein. Thus, further specific embodiment the present invention provides a method of screening for an agent for treating or preventing cancer, in particular an adenocarcinoma, which includes the steps of: contacting a test agent with the marker gene-derived polypeptide or a functional fragment thereof; detecting the binding between the polypeptide (or fragment) and the test agent; and selecting the test agent that binds to the polypeptide (or fragment). The binding of a test agent to the marker-gene derived polypeptide may be, for example, detected by immunoprecipitation using an antibody against the polypeptide.

Therefore, for the purpose for such a detection, it is preferred that the marker gene-derived polypeptide or functional fragments thereof used for the screening contains an antibody recognition site. The antibody used for the screening may be one that recognizes an antigenic region of the marker gene-derived polypeptide. Further preparation methods are known to the person skilled in the art. Alternatively, the marker gene-derived polypeptide or a functional fragment thereof may be expressed as a fusion protein including at its N- or C-terminus a recognition site (epitope) of a monoclonal antibody, whose specificity has been revealed, to the N- or C-terminus of the polypeptide. A commercially available epitope-antibody system can be used. Vectors which can express a fusion protein with, for example, β-galactosidase, maltose binding protein, glutathione S-transferase, green florescence protein (GFP), and such by the use of its multiple cloning sites are commercially available and can be used for the present invention.

Furthermore, fusion proteins containing much smaller epitopes to be detected by immunoprecipitation with an antibody against the epitopes are also known in the art (Experimental Medicine (1995) 13, 85-90). Examples include, but are not limited to, polyhistidine (His-tag), influenza aggregate HA, human c-*myc*, FLAG, Vesicular stomatitis virus glycoprotein (VSV-GP), T7 gene 10 protein (T7-tag), human simple herpes virus glycoprotein (HSV-tag), E-tag (an epitope on monoclonal phage) etc. Glutathione S-transferase (GST) is another well-established example. When GST is used as the protein to be fused with the marker gene-derived polypeptide or fragment thereof to form a fusion protein, the fusion protein can be detected either with an antibody against GST or a substance specifically binding to GST, i.e., such as glutathione (e.g., glutathione-Sepharose 4B).

In immunoprecipitation techniques, an immune complex is formed by contacting an antibody (recognizing the marker gene-derived polypeptide or a functional fragment thereof or an epitope tagged to the polypeptide or fragment) to the reaction mixture comprising the marker gene-derived polypeptide and the test agent. If the test agent has the ability to bind the polypeptide, then the formed immune complex will be composed of the marker gene-derived polypeptide, the test agent, and the antibody. On the contrary, if the test agent is devoid of such ability, then the formed immune complex only includes the marker gene-derived polypeptide and the antibody. Therefore, the binding ability of a test agent to marker gene-derived polypeptide can be examined by, for example, measuring the size of the formed immune complex. Any method for detecting the size of a substance can be used, including chromatography, electrophoresis, and such. For example, when mouse IgG antibody is used for the detection, Protein A or Protein G sepharose can be used for quantifying the immune complex formed.

Furthermore, the marker gene-derived polypeptide or a functional fragment thereof may be used for the screening of agents that bind to thereto may be bound to a carrier. Example of carriers that may be used for binding the polypeptides include insoluble polysaccharides, such as agarose, cellulose and dextran; and synthetic resins, such as polyacrylamide, polystyrene and silicon; preferably commercially available beads and plates (e.g., multi-well plates, biosensor chip, etc.) prepared from the above materials may be used. When using beads, they may be filled into a column. Alternatively, the use of magnetic beads is also known in the art, and enables to readily isolate polypeptides and agents bound on the beads via magnetism.

The binding of a polypeptide to a carrier may be conducted according to routine methods, such as chemical bonding and physical adsorption. Alternatively, a polypeptide may be bound to a carrier via antibodies specifically recognizing the protein. Moreover, binding of a polypeptide to a carrier can also be conducted by means of interacting molecules, such as the combination of avidin and biotin.

Screening methods using such carrier-bound marker gene-derived polypeptide or functional fragments thereof include, for example, the steps of contacting a test agent to the carrier- bound polypeptide, incubating the mixture, washing the carrier, and detecting and/or measuring the agent bound to the carrier. The binding may be carried out in buffer, for example, but are not limited to, phosphate buffer and Tris buffer, as long as the buffer does not inhibit the binding.

An exemplary screening method wherein such carrier-bound marker gene-derived polypeptide or fragments thereof and a composition (e.g., cell extracts, cell lysates, etc.) are used as the test agent includes affinity chromatography. For example, the marker gene-derived polypeptide may be immobilized on a carrier of an affinity column, and a test agent, containing a substance capable of binding to the polypeptides, is applied to the column. After loading the test agent, the column is washed, and then the substance bound to the polypeptide is eluted with an appropriate buffer.

A biosensor using the surface plasmon resonance phenomenon may be used as a mean for detecting or quantifying the bound agent in the present invention. When such a biosensor is used, the interaction between the marker gene-derived polypeptide and a test agent can be observed real-time as a surface plasmon resonance signal, using only a minute amount of the polypeptide and without labeling (for example, BIAcore, Pharmacia). Therefore, it is possible to evaluate the binding between the polypeptide and test agent using a biosensor such as BIAcore.

Methods of screening for molecules that bind to a specific protein among synthetic chemical compounds, or molecules in natural substance banks or a random phage peptide display library by exposing the specific protein immobilized on a carrier to the molecules, and methods of high-throughput screening based on combinatorial chemistry techniques to isolate not only proteins but chemical compounds are also well-known to those skilled in the art. These methods can also be used for screening agents (including agonist and antagonist) that bind to the marker gene-derived protein or fragments thereof.

When the test agent is a protein, for example, West-Western blotting analysis (Skolnik, E. et al. (1991) Cell 65 83-90) can be used for the present method. Specifically, a protein binding to the marker gene-derived polypeptide can be obtained by preparing first a cDNA library is prepared from cells, tissues, organs, or cultured cells (e.g., NSCLC) expected to express at least one protein binding to the marker gene-derived polypeptide using a phage vector (e.g., ZAP), expressing the proteins encoded by the vectors of the cDNA library on LB-agarose, fixing the expressed proteins on a filter, reacting the purified and labeled marker gene-derived polypeptide with the above filter, and detecting the plaques expressing proteins to which the marker gene-derived polypeptide has bound according to the label of the marker gene-derived polypeptide.

Labeling substances such as radioisotope, enzymes (e.g., alkaline phosphatase, horseradish peroxidase, β-galactosidase, β-glucosidase), fluorescent substances and biotin/avidin, may be used for the labeling of marker gene-derived polypeptide in the present method. When the protein is labeled with radioisotope, the detection or measurement can be carried out by liquid scintillation. Alternatively, when the protein is labeled with an enzyme, it can be detected or measured by adding a substrate of the enzyme to detect the enzymatic change of the substrate, such as generation of color, with absorptiometer. Further, in case where a fluorescent substance is used as the label, the bound protein may be detected or measured using fluoro-photometer.

Moreover, the marker gene-derived polypeptide bound to the protein can be detected or measured by utilizing an antibody that specifically binds to the marker gene-derived polypeptide, or a peptide or polypeptide (for example, GST) that is fused to the marker gene-derived polypeptide. In case of using an antibody in the present screening, the antibody is preferably labeled with one of the labeling substances mentioned above, and detected or measured based on the labeling substance.

Alternatively, the antibody against the marker gene-derived polypeptide may be used as a primary antibody to be detected with a secondary antibody that is labeled with a labeling substance. Furthermore, the antibody bound to the marker gene-derived polypeptide in the present screening may be detected or measured using protein G or protein A column.

Alternatively, in another embodiment of the screening method of the present invention, two-hybrid system utilizing cells may be used. In two-hybrid system, marker gene-derived polypeptide or a fragment thereof is fused to the SRF-binding region or GAL4-binding region and expressed in yeast cells. A cDNA library is prepared from cells expected to express at least one protein binding to the marker gene-derived polypeptide, such that the library, when expressed, is fused to the VP 16 or GAL4 transcriptional activation region. The cDNA library is then introduced into the above yeast cells and the cDNA derived from the library is isolated from the positive clones detected (when a protein binding to the marker gene-derived polypeptide is expressed in the yeast cells, the binding of the two activates a reporter gene, making positive clones detectable). A protein encoded by the cDNA can be prepared by introducing the cDNA isolated above to E. coli and expressing the protein. As a reporter gene, for example, Ade2 gene, lacZ gene, CAT gene, luciferase gene and such can be used in addition to the HIS3 gene.

The agent identified by this screening is a candidate for agonists or antagonists of the marker gene-derived polypeptide. The term "agonist" refers to molecules that activate the function of the polypeptide by binding thereto. On the other hand, the term "antagonist" refers to molecules that inhibit the function of the polypeptide by binding thereto. Moreover, an agent isolated by this screening as an antagonist is a candidate that inhibits the in vivo interaction of the marker gene-derived polypeptide with molecules (including nucleic acids (RNAs and DNAs) and proteins).

Furthermore, agents that suppress or inhibit the biological function of the translational product of the marker gene(s) are considered to serve as candidates for treating or preventing cancer, in particular an adenocarcinoma. Thus, the present invention also provides a method of screening for a compound for treating or preventing adenocarcinoma using the marker gene-derived polypeptide or fragments thereof including the steps: (a) contacting a test agent with the marker gene-derived polypeptide or a functional fragment thereof; and (b) detecting the biological activity of the polypeptide or fragment of step (a). Any polypeptide can be used for the screening so long as it has one biological activity of the marker gene-derived polypeptide that can be used as an index in the present screening method. Since the marker gene-derived polypeptide has the activity of promoting cell proliferation of cancer cells, biological activities of the marker gene-derived polypeptide that can be used as an index for the screening include such cell-proliferating activity of the marker gene-derived polypeptide. For example, a marker gene-derived polypeptide can be used and polypeptides functionally equivalent thereto including functional fragments thereof can also be used. Such polypeptides may be expressed endogenously or exogenously.

When the biological activity to be detected in the present method is cell proliferation, it can be detected, for example, by preparing cells which express the marker gene-derived polypeptide or a functional fragment thereof, culturing the cells in the presence of a test agent, and determining the speed of cell proliferation, measuring the cell cycle and such, as well as by detecting wound-healing activity, conducting a Matrigel invasion assay and measuring the colony forming activity. According to an aspect of the present invention, the screening further includes, after the above step (b), the step of: c) selecting the test agent that suppresses the biological activity of the polypeptide as compared to the biological activity detected in the absence of the test agent.

The agent isolated by this screening is a candidate for an antagonist of the marker gene-derived polypeptide, and thus, is a candidate that inhibits the in vivo interaction of the polypeptide with molecules (including nucleic acids (RNAs and DNAs) and proteins).

Furthermore, agents that may be used in the treatment or prevention of cancers can be identified through screenings that use the expression levels of the marker genes as indices. In the context of the present invention, such screening may include, for example, the following steps: a) contacting a test agent with a cell expressing a marker gene; b) detecting the expression level of the marker gene; and c) selecting the test agent that reduces the expression level of the marker gene as compared to a level detected in the absence of the test agent.

An agent that inhibits the expression of the marker gene or the activity of its gene product can be identified by contacting a cell expressing the marker gene with a test agent and then determining the expression level of the marker gene. Naturally, the identification may also be performed using a population of cells that express the gene in place of a single cell. A decreased expression level detected in the presence of an agent as compared to the expression level in the absence of the agent indicates the agent as being an inhibitor of the marker gene, suggesting the possibility that the agent is useful for inhibiting cancer, thus a candidate agent to be used for the treatment or prevention of cancer.

The expression level of a gene can be estimated by methods well known to one skilled in the art. The expression level of the marker gene can be, for example, determined as described herein above. The cell or the cell population used for such an identification may be any cell or any population of cells so long as it expresses the marker gene. For example, the cell or population may be or contain an epithelial cell derived from a tissue. Alternatively, the cell or population may be or contain an immortalized cell derived from an adenocarcinoma cell. Cells expressing the marker gene include, for example, cell lines established from cancers. Furthermore, the cell or population may be or contain a cell, which has been transfected with marker genes

The present method permits the screening of various agents mentioned above and is particularly suited for identifying functional nucleic acid molecules including antisense RNA, siRNA, etc.

In a further preferred embodiment, the present invention relates to a pharmaceutical composition comprising any one or more agents selected from the group consisting of: an antisense nucleic acid construct, an siRNA, a riboyzme or an antibody directed against or a dominant negative polypeptide variant of any one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397). Preferably, the pharmaceutical composition comprises agents identified and selected in accordance with the herein above-described methods and screening approaches. The compositions may be used as pharmaceuticals for human beings and other mammals, e.g., mice, rats, guinea pigs, rabbits, cats, dogs, sheep, pigs or cattle.

In the context of the present invention, suitable pharmaceutical formulations for the active ingredients of the present invention detailed below (including screened agents, antisense nucleic acids, siRNA, antibodies, etc.) include those suitable for oral, rectal, nasal, topical (including buccal and sub-lingual), vaginal or parenteral (including intramuscular, subcutaneous and intravenous) administration, or for administration by inhalation or insufflation. Preferably, administration is intravenous. The formulations are optionally packaged in discrete dosage units.

All these pharmaceutical formulations are well established in the art (see, e.g., Gennaro, A.L. and Gennaro, A.R. (2000) Remington: The Science and Practice of Pharmacy, 20th Ed., Lippincott Williams & Wilkins, Philadelphia, PA; Crowder, T.M. et al. (2003) A Guide to Pharmaceutical Particulate Science. Interpharm/CRC, Boca Raton, FL; Niazi, S.K. (2004) Handbook of Pharmaceutical Manufacturing Formulations, CRC Press, Boca Raton, FL).

Pharmaceutical formulations suitable for oral administration include capsules, microcapsules, cachets and tablets, each containing a predetermined amount of active ingredient. Suitable formulations also include powders, elixirs, granules, solutions, suspensions and emulsions. The active ingredient is optionally administered as a bolus electuary or paste. Alternatively, according to needs, the pharmaceutical composition may be administered non-orally, in the form of injections of sterile solutions or suspensions with water or any other pharmaceutically acceptable liquid. For example, the active ingredients of the present invention can be mixed with pharmaceutically acceptable carriers or media, specifically, sterilized water, physiological saline, plant-oils, emulsifiers, suspending agents, surfactants, stabilizers, flavoring agents, excipients, vehicles, preservatives, binders, and such, in a unit dose form required for generally accepted drug implementation. The amount of active ingredient contained in such a preparation makes a suitable dosage within the indicated range acquirable. Examples of additives that can be admixed into tablets and capsules include, but are not limited to, binders, such as gelatin, corn starch, tragacanth gum and arabic gum; excipients, such as crystalline cellulose; swelling agents, such as corn starch, gelatin and alginic acid; lubricants, such as magnesium stearate; sweeteners, such as sucrose, lactose or saccharin; and flavoring agents, such as peppermint, Gaultheria adenothrix oil and cherry. A tablet may be made by compression or molding. Compressed tablets may be prepared by compressing in a suitable machine the active ingredients in a free-flowing form such as powder or granules, optionally mixed with a binder, lubricant, inert diluent, lubricating, surface active or dispersing agent. Molded tablets may be made via molding in a suitable machine a mixture of the powdered compound moistened with an inert liquid diluent. The tablets may be coated according to methods well known in the art. The tablets may optionally be formulated so as to provide slow or controlled release of the active ingredient in vivo. A package of tablets may contain one tablet to be taken on each of the month. Furthermore, when the unit-dosage form is a capsule, a liquid carrier, such as oil, can be further included in addition to the above ingredients.

Oral fluid preparations may be in the form of, for example, aqueous or oily suspensions, solutions, emulsions, syrups or elixirs, or may be presented as a dry product for reconstitution with water or other suitable vehicle prior to use. Such liquid preparations may contain conventional additives such as suspending agents, emulsifying agents, non-aqueous vehicles (which may include edible oils) or preservatives.

Formulations for parenteral administration include aqueous and non-aqueous sterile injection solutions which may contain anti-oxidants, buffers, bacteriostatic compounds and solutes which render the formulation isotonic with the blood of the intended recipient; and aqueous and non-aqueous sterile suspensions which may include suspending agents and thickening agents. The formulations may be presented in unit dose or multi-dose containers, for example sealed ampoules and vials, and may be stored in a freeze-dried (lyophilized) condition requiring only the addition of the sterile liquid carrier, for example, saline, water-for-injection, immediately prior to use. Alternatively, the formulations may be presented for continuous infusion.

Extemporaneous injection solutions and suspensions may be prepared from sterile powders, granules and tablets of the kind previously described.

Moreover, sterile composites for injection can be formulated following normal drug implementations using vehicles, such as distilled water, suitable for injection. Physiological saline, glucose, and other isotonic liquids, including adjuvants, such as D-sorbitol, D-mannose, D-mannitol, and sodium chloride, can be used as aqueous solutions for injection. These can be used in conjunction with suitable solubilizers, such as alcohol, for example, ethanol; polyalcohols, such as propylene glycol and polyethylene glycol; and non-ionic surfactants, such as Polysorbate 80 (TM) and HCO-50. Sesame oil or soy-bean oil can be used as an oleaginous liquid, which may be used in conjunction with benzyl benzoate or benzyl alcohol as a solubilizer, and may be formulated with a buffer, such as phosphate buffer and sodium acetate buffer; a pain-killer, such as procaine hydrochloride; a stabilizer, such as benzyl alcohol and phenol; and/or an anti-oxidant. A prepared injection may be filled into a suitable ampoule. Formulations for rectal administration include suppositories with standard carriers such as cocoa butter or polyethylene glycol. Formulations for topical administration in the mouth, for example, buccally or sublingually, include lozenges, which contain the active ingredient in a flavored base such as sucrose and acacia or tragacanth, and pastilles including the active ingredient in a base such as gelatin, glycerin, sucrose or acacia. For intra-nasal administration of an active ingredient, a liquid spray or dispersible powder or in the form of drops may be used. Drops may be formulated with an aqueous or non-aqueous base also including one or more dispersing agents, solubilizing agents or suspending agents. For administration by inhalation the compositions are conveniently delivered from an insufflator, nebulizer, pressurized packs or other convenient means of delivering an aerosol spray. Pressurized packs may include a suitable propellant such as dichlorodifluoromethane, trichlorofluoromethane, dichiorotetrafluoroethane, carbon dioxide or other suitable gas. In the case of a pressurized aerosol, the dosage unit may be determined by providing a valve to deliver a metered amount.

Alternatively, for administration by inhalation or insufflation, the compositions may take the form of a dry powder composition, for example, a powder mix of an active ingredient and a suitable powder base such as lactose or starch. The powder composition may be presented in unit dosage form in, for example, capsules, cartridges, gelatin or blister packs from which the powder may be administered with the aid of an inhalator or insufflators.

Other formulations include implantable devices and adhesive patches; which release a therapeutic agent.

When desired, the above-described formulations, adapted to give sustained release of the active ingredient, may be employed. The pharmaceutical compositions may also contain other active ingredients such as antimicrobial agents, immunosuppressants or preservatives.

It should be understood that in addition to the ingredients particularly mentioned above, the formulations of this invention may include other agents conventional in the art having regard to the type of formulation in question, for example, those suitable for oral administration may include flavoring agents.

The present invention provides compositions for treating or preventing cancers including any of the agents selected by the above-described screening methods of the present invention.

An agent identified by a method of the present invention can be directly administered or can be formulated into a dosage form according to any conventional pharmaceutical preparation method detailed above.

In a particularly preferred embodiment a pharmaceutical composition as defined herein above is used for the prevention and/or treatment of adenocarcinoma.

In a further preferred embodiment an antisense nucleic acid construct, an siRNA, a riboyzme or an antibody directed against or a dominant negative polypeptide variant of any one of the marker genes *RNPC1* (Genbank accesion # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *TH1L* (Genbank accession # NM_016397) is used for the preparation of a pharmaceutical composition for the prevention and/or treatment of an adenocarcinoma.

"Antisense nucleic acids" in the context of the present invention corresponding to the nucleotide sequence of any one of the marker gene can of the present invention be used to reduce the expression level of the gene, which is up-regulated in various cancerous cells, are useful for the treatment of cancer, in particular adenocarcinoma, and thus are also encompassed by the present invention. An antisense nucleic acid acts by binding to the nucleotide sequence of the marker gene, or mRNAs corresponding thereto, thereby inhibiting the transcription or translation of the gene, promoting the degradation of the mRNAs, and/or inhibiting the expression of the protein encoded by the gene. Thus, as a result, an antisense nucleic acid inhibits the marker gene-derived protein to function in the cancerous cell. Herein, the phrase "antisense nucleic acids" refers to "classical" antisense-technology, that is, nucleotides that typically have more than about 25, more than 50 or more than 100 nucleotides in length that specifically hybridize to a target sequence and includes not only nucleotides that are entirely complementary to the target sequence but also that includes mismatches of one or more nucleotides. For example, the antisense nucleic acids of the present invention include polynucleotides that have a homology of at least 70% or higher, preferably of at least 80% or higher, more preferably of at least 90% or higher, even more preferably of at least 95% or higher over a span of at least 15 continuous nucleotides of any of the marker genes of the present invention or the complementary sequence thereof. Algorithms known in the art can be used to determine such homology.

The term "siRNA" refers to a particular type of antisense-molecules, namely small inhibitory RNA duplexes that induce the RNA interference (RNAi) pathway. These molecules can vary in length (generally 18-30 base pairs, preferably 21-23 base pairs) and contain varying degrees of complementarity to their target mRNA in the antisense strand. Some, but not all, siRNA have unpaired overhanging bases on the 5' or 3' end of the sense strand and/or the antisense strand. The term "siRNA" includes duplexes of two separate strands, as well as single strands that can form hairpin structures comprising a duplex region. Methods for designing suitable siRNAs directed to a given target nucleic acid are established in the art (cf., for example, Elbashir S.M. et al. (2001) Genes Dev. 15, 188-200)

Antisense nucleic acids (including siRNAs) of the present invention act on cells producing proteins encoded by the marker gene by binding to the DNA or mRNA of the gene, inhibiting their transcription or translation, promoting the degradation of the mRNA, and inhibiting the expression of the protein, finally inhibiting the protein to function.

Antisense nucleic acids of the present invention can be made into an external preparation, such as a liniment or a poultice, by admixing it with a suitable base material which is inactive against the nucleic acids.

Also, as needed, the antisense nucleic acids of the present invention can be formulated into tablets, powders, granules, capsules, liposome capsules, injections, solutions, nose-drops and freeze-drying agents by adding excipients, isotonic agents, solubilizers, stabilizers, preservatives, pain-killers, and such. An antisense-mounting medium can also be used to increase durability and membrane-permeability. Examples include, but are not limited to, liposomes, poly-L-lysine, lipids, cholesterol, lipofectin, or derivatives of these. These can be prepared by following known methods.

The antisense nucleic acids of the present invention inhibit the expression of the marker gene-derived protein and are useful for suppressing the biological activity of the protein. In addition, expression-inhibitors, including antisense nucleic acids of the present invention, are useful in that they can inhibit the biological activity of the marker gene-derived protein.

The antisense nucleic acids of present invention include modified oligonucleotides. For example, thioated oligonucleotides may be used to confer nuclease resistance to an oligonucleotide.

In a further specific aspect the present invention relates to the use of antibodies against a protein encoded by the marker gene, or fragments of the antibodies. An antibody may be modified by conjugation with a variety of molecules, such as polyethylene glycol (PEG). The present invention includes such modified antibodies. The modified antibody can be obtained by chemically modifying an antibody. Such modification methods are conventional in the field. Alternatively, the antibody used for the present invention may be a chimeric antibody having a variable region derived from a non-human antibody against the marker gene-derived polypeptide and a constant region derived from a human antibody, or a humanized antibody, composed of a complementarity determining region (CDR) derived from a non-human antibody, a frame work region (FR) and a constant region derived from a human antibody. Such antibodies can be prepared by using known technologies. Humanization can be performed by substituting rodent CDRs or CDR sequences for the corresponding sequences of a human antibody. Accordingly, such humanized antibodies are chimeric antibodies, wherein substantially less than an intact human variable domain has been substituted by the corresponding sequence from a non-human species. Complete human antibodies including human variable regions in addition to human framework and constant regions can also be used. Such antibodies can be produced using various techniques known in the art. For example in vitro methods involve use of recombinant libraries of human antibody fragments displayed on bacteriophage.

Similarly, human antibodies can be made by introducing human immunoglobulin loci into transgenic animals, e.g., mice in which the endogenous immunoglobulin genes have been partially or completely inactivated. When the obtained antibody is to be administered to the human body (antibody treatment), a human antibody or a humanized antibody is preferable for reducing immunogenicity.

Antibodies obtained as above may be purified to homogeneity. For example, the separation and purification of the antibody can be performed according to separation and purification methods used for general proteins. For example, the antibody may be separated and isolated by the appropriately selected and combined use of column chromatographies, such as affinity chromatography, filter, ultrafiltration, salting-out, dialysis, SDS polyacrylamide gel electrophoresis, isoelectric focusing, and others (Antibodies: A Laboratory Manual. Ed Harlow and David Lane, Cold Spring Harbor Laboratory (1988)), but are not limited thereto. A protein A column and protein G column can be used as the affinity column. Exemplary protein A columns to be used include, for example, Hyper D, POROS, and Sepharose F F. (Pharmacia). Exemplary chromatography, with the exception of affinity includes, for example, ion- exchange chromatography, hydrophobic chromatography, gel filtration, reverse-phase chromatography, adsorption chromatography, and the like (Strategies for Protein Purification and Characterization: A Laboratory Course Manual. Ed Daniel R. Marshak et al, Cold Spring Harbor Laboratory Press (1996)). The chromatographic procedures can be carried out by liquid-phase chromatography, such as HPLC and FPLC.

While the above invention has been described with respect to some of its preferred embodiments, this is in no way to limit the scope of the invention. The person skilled in the art is clearly aware of further embodiments and alterations to the previously described embodiments that are still within the scope of the present invention.

### EXAMPLES

### Example 1

### Tumor samples

Forty-one formalin-fixed and paraffin-embedded progressed adenomas (with a focus of adenocarcinoma present, also referred as malignant polyps) collected from the tissue archive of the department of pathology at the VU University Medical Center (VUmc), Amsterdam, the Netherlands, and 73 prospectively collected snap-frozen colorectal tumor samples (37 non-progressed adenomas and 36 adenocarcinomas) were investigated. All samples were used in compliance with the institution's ethical regulations.

The 41 progressed adenomas corresponded to 19 females and 18 males (three patients presented more than one lesion). Mean age was 67 (range 45-86). From these, adenoma and adenocarcinoma components were analyzed separately adding to a total of 82 archival samples (41 x 2).

The 73 frozen specimens corresponded to 31 females and 34 males (six patients had multiple tumors). Mean age was 69 (range 47-89). All histological sections were evaluated by a pathologist. Array CGH was performed on both sets of samples while expression microarrays were performed on the frozen samples only.

### DNA and RNA isolation

DNA from paraffin was obtained as described previously (Weiss, M.M. et al. (1999) Mol. Pathol. 52, 243-251). RNA and DNA from snap-frozen tissues were isolated using TRIzol (Invitrogen, Breda, NL) following the supplier's instructions with some modifications, described on http://www.english.vumc.nl/afdelingen/microarrays. Isolated RNA was subjected to purification using RNeasy Mini Kit (Qiagen, Venlo, NL). RNA and DNA concentrations and purities were measured on a Nanodrop ND-1000 spectrophotometer (Isogen, IJsselstein, NL) and integrity was evaluated on a 1% agarose ethidium bromide-stained gel.

### Array CGH

A BAC/PAC array platform was used as described elsewhere (Carvalho, B. et al. (2006) Cell. Oncol. 28, 283-294). Arrays were scanned (Agilent DNA Microarray scanner G2505B- Agilent Technologies, Palo Alto, USA) and Imagene 5.6 software (Biodiscovery Ltd, Marina del Rey, California) was used for automatic feature extraction with default settings. Local background was subtracted from the signal median intensities of both test and reference DNA. The median of the triplicate spots was calculated for each BAC clone and log₂ ratios (tumor/normal) were normalized by subtraction of the mode value of BAC clones on chromosomes 1-22 (UCSC July2003 freeze of the Human Golden Path - NCBI Build 34). Clones with standard deviation of the intensity of the three spots greater than 0.2 and with more than 20% missing values were excluded.

### Expression microarrays

The Human Release 2.0 oligonucleotide library, containing 60-mer oligonucleotides representing 28830 unique genes, designed by Compugen (San Jose, CA, USA) was obtained from Sigma-Genosys (Zwijndrecht, NL). Printing of slides was done as described elsewhere (Muris, J.J. et al. (2007) Br. J. Haematol. 136 38-47). Tumor RNA (30µg) was hybridized against Universal Human reference (Stratagene, Amsterdam, NL). cDNA labeling and hybridization procedures are described elsewhere (Muris, J.J. et al.., *supra*). Scanning of arrays and feature extraction were performed as described above. Overall quality of experiments was judged on MA-plots of intensities of raw data. Normalization was done with TIGR Midas (http://www.tm4.org/midas.html), using "Lowess" correction (Quackenbush, J. (2002) Nat. Genet. 32, Suppl. 496-501) or with "Median" normalization and implemented in the maNorm function (Marray R bioconductor package), with identical results. Inter-array normalization was also performed. Low intensity values were replaced by the intensity value of 50. Genes with more than 20% missing values were excluded.

Array CGH and expression microarray data sets are available at Gene Expression Omnibus (GEO) http://www.ncbi.nlm.nih.gov/geo/ (Edgar, R. et al. (2002) Nucleic Acids Res. 30, 207-210); accession number GSE8067.

### Microarray data analysis

Below, the steps of data analysis are discussed for array CGH data, expression data and integrative analysis. To account for multiple testing, either a False Discovery Rate (FDR) correction was applied to the p-values, or a very stringent p-value cut-off was used.

### Array CGH data

To segment DNA copy number alterations, a smoothing algorithm - "aCGH-Smooth" was applied (Jong, K. et al. (2004) Bioinformatics 20, 3636-3637). Smoothed log₂ ratios of -0.15 and 0.15 were used as thresholds to define gains and losses (99% confidence intervals) obtained for 15 normal-to-normal hybridizations. Only gains and losses covering at least three consecutive BAC clones were included. Amplifications were called when log₂ ratios exceeded 1.0. DNA copy number data were stored in the ArrayCGHbase (Menten, B. (2005) BMC Bioinformatics 6, 124) (http://arraydb.vumc.nl/arrayCGHbase). Median absolute deviation (MAD) was determined for each case as a quality control. Cases with MAD ≥ 0.2 were excluded. Array CGH profiles were visualized in ArrayCGHbase.

Supervised analysis, comparing two groups, was done using CGHMultiArray (van de Wiel, M.A. et al. (2005) Bioinformatics 21, 3193-3194). For analysis of paired samples (adenoma and adenocarcinoma components within progressed adenomas) an adapted version of CGHMultiArray was used, based on the Wilcoxon sign-rank test corrected for ties. Reported p-values are adjusted for multiple testing (FDR), unless stated otherwise.

For defining the most frequent smallest regions of overlap (SRO) for gains on 20q, throughout all cases, STAC (Significance Testing for Aberrant Copy-number) was used (Diskin, S.J. et al. (2006) Genome Res. 16, 1149-1158).

### Microarray expression data

As all hybridizations were performed against a common reference, all comparisons were relative between colorectal adenomas and adenocarcinomas.

Supervised analysis for comparing carcinomas and adenomas was done using the Wilcoxon signed rank test, and a modified version of this test- total Thas score (http://www.cvstat.ugent.be/index.php?page=techrep/techrep.htm) that is powerful when the distributions of the expression levels of both groups do not differ over the whole range of expression levels. This occurs when not all cases in the adenocarcinomas and adenomas groups have differentially expressed genes, but differences rather appear in subpopulations. Genes were considered as differentially expressed when a Wilcoxon test p-value <1e-5 and a Thas p-value < 0.05, corresponding to a FDR < 0.05.

To disclose genes which expression is influenced by 20q gain, tumors with and without a 20q gain were compared. Gene expression was regressed on copy number count using a linear model.

To evaluate the discriminatory power of candidate genes for classifying adenomas *versus* adenocarcinomas, a stepwise linear discriminant analysis with leave one out cross validation was performed on mRNA expression data (SPSS 15.0 for Windows, SPSS Inc, Chicago, IL, USA).

### Integration of copy number and expression data

ACE-it (Array CGH Expression integration tool) was applied to test whether gene dosage affects RNA expression (van Wieringen, W.N. et al. (2006) Bioinformatics 22, 1919-1920). Only genes on chromosome 20 are presented. We used a cut-off value of 0.15 for gains and losses, a default group value of 9 and a FDR ≤ 0.10.

### Quantitative RT-PCR

RNA (1 µg) was treated with DNase I and reverse transcribed to cDNA using oligo(dT)₂₀ Primer with Superscript II reverse transcriptase (Invitrogen, Breda, NL).

qRT-PCR was performed in duplicate on 15 adenomas and 15 adenocarcinomas for six candidate genes. A master mix was prepared with 12.5 µl of SYBR Green PCR master mix (Applied Biosystems, Nieuwerkerk a/d IJssel, NL), 0.5 µM of each primer in 22.5 µl. cDNA (25ng in 2.5µl) was added to the mix. Reactions were performed in a 7300 Real-time PCR System (Applied Biosystems, Nieuwerkerk a/d IJssel, NL). Amplification conditions comprised a denaturation step at 95°C for 10' and 50 cycles at 95°C for15" and annealing temperature for 1' (Supplementary Table 1). Relative expression levels were determined following the 2ΔΔACt method (Livak, K.J. and Schmittgen, T.D. (2001) Methods 25, 402-408) using β*2M* (beta-2-microglobulin gene) as a reference. This gene was previously demonstrated not to differ in expression between adenomas and adenocarcinoma (Dydensborg, A.B. et al. (2006) Am. J. Physiol. Gastrointest. Liver Physiol. 290, G1067-G1074).

### Immunohistochemistry on tissue microarrays (TMAs)

A tissue microarray (TMA) was constructed with 57 tumors (32 adenomas and 25 adenocarcinomas) of which array CGH and/or expression microarray data were available. Of each tumor three cores from different locations within the tumor were included in the array. A 4 µm section of the array was used for immunohistochemistry. After deparaffination in xylene, and rehydration through graded alcohol to water, endogenous peroxidase was blocked with hydrogen peroxide (0.3% H₂O₂/methanol) for 25 min. Antigen retrieval was done by autoclaving in citrate buffer (10 mM; pH 6.0). Primary Aurora A monoclonal antibody NCL-L-AK2 from Novocastra Laboratories was incubated overnight at 4°C in a dilution of 1:50. The secondary antibody - K4006, mouse, from Envision kit (DAKO) was incubated for 30 min at room temperature. Counterstaining was done with Mayer's hematoxylin. Incubation without primary antibody was used as negative control. Colorectal cancer cell line Caco-2, which has a 20q gain and is known to express Aurora A, was used as positive control. Caco-2 cells were fixed and paraffin embedded, sections of which were taken along in the same run of immunohistochemistry as the tissue microarray was processed. Caco-2 produced strong nuclear, mostly along with cytoplasmic, staining in > 75% of tumor cells and this pattern was taken as reference for intense staining.

Next, the spectrum of staining in the respective cores on the TMA was surveyed in terms of intensity and positive nuclei. Only staining in tumor cells (i.e. either adenoma or adenocarcinoma cells) was considered. Cores of the TMA typically contained 4 to 17 crypts with in every crypt > 100 cells which all were evaluated. Basically, three staining patterns were seen; no staining at all, strong staining comparable to that observed in Caco-2 cells, and an intermediate pattern that showed positive staining, but clearly less intense than in Caco-2 cells. The intensity of staining was taken as most important parameter. In pattern 2, typically 50% to >75% of nuclei showed intense staining, while in pattern 1 typically 25% to >75% of nuclei showed weak staining. For score 0, no more than a scattered weakly positive cell was tolerated. Based on evaluation of up to three cores by two independent observers, a score ranging from 0 to 2 was assigned per tumor, with score 0 corresponding to no signal, score 2 corresponding to the strong signal that was observed in the positive control Caco-2 and score 1 for an intermediate intensity staining. In case of disagreement between observers, a third observer was consulted and the majority score was noted.

Cochran-Armitage test analysis was performed to compare protein expression with lesion type (adenoma, carcinoma). Jonckheere-Terpstra test was performed to compare protein expression with log₂ratios (expression data). Both tests make explicit use of the ordinality of the protein levels of expression. Differences were considered significant when p< 0.05.

### Example 2 - Delimiting gained regions on 20q

41 progressed adenomas, which were previously studied by classical CGH, were analyzed by array CGH. The adenoma and adenocarcinoma components of these samples were tested separately. Gain of 20q occurred in more than 60% of the cases (Figure 1A, 1B; Supplementary Figure 1A). The pattern of copy number changes did not differ between adenoma and adenocarcinoma components (as determined by CGHMultiArray), although sometimes showed lower amplitudes in the adenoma component (Figure 1A and 1B).

Next, the DNA copy number status of 37 non-progressed adenomas and 36 adenocarcinomas was analyzed. From these 73 tumors, 67 (34 adenomas and 33 adenocarcinomas) showed high quality genomic profiles with MAD values < 0.2, giving an 8% drop-out. In these 67 tumors, chromosome 20 gain occurred in less than 15% of the adenomas but in more than 60% of the carcinomas (p < 0.00001, as determined by CGHMultiArray), mostly affecting either all of chromosome 20 or the q-arm only, similar to the progressed adenomas (Figure 1C, 1D; Supplementary Figure 1B).

To determine the most relevant regions within 20q harboring putative oncogenes with a role in colorectal adenoma to adenocarcinoma progression, STAC (Diskin, S.J. et al., *supra*) was applied to the combined set of paraffin-embedded malignant polyps (n = 41) and frozen carcinomas (n = 33). This revealed 3 relevant regions of aberrant copy gains on 20q, one spanning 4 Mb (32-36Mb), one spanning 3 Mb (56-59Mb), and the third one spanning 2Mb (61-64Mb) (Figure 2). These three regions (smallest regions of overlap - SROs) contained 80, 35, and 94 known genes, respectively.

### Example 3 - Identification of differentially expressed genes

Microarray expression analysis on the 37 non-progressed adenomas and 36 adenocarcinomas of which snap-frozen material was available were performed. High quality expression array data were obtained from 68 cases (37 adenomas and 31 adenocarcinomas, 7% drop-out).

Supervised data analysis for identifying putative oncogenes on 20q, was done in two different ways; we compared carcinomas to adenomas, and we compared tumors with 20q gain to tumors without 20q gain. The first approach revealed genome-wide 122 up-regulated genes and 219 down-regulated genes (a total of 341 differentially expressed genes), in carcinomas when compared to adenomas (Wilcoxon test p-value < 1e-5 (FDR < 0.05) and Thas p-value < 0.05). Of these 122 up-regulated genes, 14 map at chromosome 20q (Table 1). For the second approach, only tumors (adenomas and adenocarcinomas) that had both array CGH data and expression data available (n = 64) were included. As a preselection, genes differentially expressed (both up and down) between carcinomas and adenomas were used that are involved in progression, using a less stringent cut-off (Thas p-value < 0.05). Thereby, 127 genes were identified genome-wide out of 931 differentially expressed genes (regression analysis; FDR ≤0.1), whose expression levels are influenced by the occurrence of 20q gain. Of these 127 genes, 21 are mapped at 20q (Table 2).

Nine genes common to these two approaches emerged, namely *TPX2, C20orf24, AURKA, RNPC1, TH1L, ADRM1, C20orf20, TCFL5* and *C20orf11.*

**Table 1. Genes significantly up-regulated in adenocarcinomas, when compared to adenomas, mapping at 20q (Wilcoxon ranking p-value < 1e-5 (i.e. FDR < 0.05) and Thas p-value < 0.05), ordered by chromosomal position (location in bp according to Freeze July 2003; NCBI Build 34) with HUGO gene symbols and GenBank accession ID.**

| **Gene symbol** | **GenBank Accession #** | **Location (bp position)** | **Wilcoxon p-value** | **Thas p-value** |
|---|---|---|---|---|
| *C20orf1(TPX2)* | NM_012112 | 31103374 | 2E-06 | 8E-05 |
| *MYRL2* | NM_006097 | 35859501 | 5E-06 | 4E-05 |
| *C20orf24 (RIP5)* | NM_018840 | 35923014 | 2E-07 | 2E-05 |
| *TOMM34* | NM_006809 | 44265329 | 8E-08 | 0 |
| *RBPSUHL* | NM_014276 | 44626010 | 2E-07 | 6E-06 |
| *BCAS4* | NM_017843 | 50138063 | 2E-06 | 6E-05 |
| *AURKA (STK6)* | NM_003600 | 55641283 | 4E-10 | 0 |
| *FLJ37465 (BMP7)* | AK094784 | 56477906 | 1 E-09 | 0 |
| *RNPC1* | NM_017495 | 56660843 | 8E-07 | 7E-05 |
| *TH1L* | NM_016397 | 58253070 | 1E-06 | 1E-05 |
| *ADRM1* | NM_007002 | 61566389 | 9E-07 | 8E-05 |
| *C20orf20* | NM_018270 | 62156238 | 9E-09 | 0 |
| *TCFL5* | NM_006602 | 62211152 | 2E-09 | 0 |
| *C20orf11* | NM_017896 | 62299593 | 4E-07 | 0 |

**Table 2. Genes significantly up-regulated in adenocarcinomas, when compared to adenomas, mapping at 20q, which expression is related to the 20q gain (FDR ≤ 0.10), ordered by chromosomal position (Location in bp according to Freeze July 2003; NCBI Build 34) with HUGO gene symbols and GenBank accession ID.**

| **Gene symbol** | **GenBank accession #.** | **Location (bp position)** | **FDR** |
|---|---|---|---|
| *HM13* | NM_030789 | 30874805 | 0.03 |
| *C20orf1 (TPX2)* | NM_012112 | 31103374 | 0.03 |
| *CDC91L1* | NM_080476 | 33922394 | 0.02 |
| *C20orf44* | NM_018244 | 34608051 | 0.07 |
| *DLGAP4* | NM_014902 | 35761669 | 0.05 |
| *TGIF2* | NM_021809 | 35897616 | 0.003 |
| *C20orf24 (RIP5)* | NM_018840 | 35923014 | 0.0006 |
| *YWHAB* | NM_014052 | 44210177 | 0.0002 |
| *UBE2C* | NM_007019 | 45128792 | 0.01 |
| *DPM1* | NM_003859 | 50248672 | 0.000001 |
| *NFATC2* | AK025758 | 50769018 | 0.003 |
| *AURKA (STK6)* | NM_003600 | 55641283 | 0.02 |
| *RNPC1* | NM_017495 | 56660843 | 0.04 |
| *TH1L* | NM_016397 | 58253070 | 0.007 |
| *ADRM1* | NM_007002 | 61566389 | 0.05 |
| *SLCO4A1* | NM_016354 | 62015102 | 0.08 |
| *C20orf20* | NM_018270 | 62156238 | 0.04 |
| *TCFL5* | NM_006602 | 62211152 | 0.03 |
| *C20orf11* | NM_017896 | 62299593 | 0.0009 |
| *C20orf59* | NM_022082 | 62323360 | 0.007 |
| *PRPF6* | NM_012469 | 63364789 | 0.03 |

### Example 4 - Integration of array CGH and expression data

BAC array CGH data were related to oligonucleotide expression array data, independently of adenoma or adenocarcinoma status, using a dedicated integration tool called ACEit (van Wieringen, W.N. et al., *supra*). A list of 151 genes located at chromosome 20 was obtained, for which gene dosage affected expression levels (FDR ≤ 0.1), 120 of which are on the q-arm (Supplementary Table 2). Combining this information with the results of the two supervised approaches for expression data analysis (adenocarcinoma *versus* adenoma and 20q gain *versus* no-20q gain), seven genes were shared (Figure 3). For these genes, *C20orf24, AURKA, RNPC1, THIL, ADMR1, C20orf20,* and *TCFL5*, combined box plots with dot plots of mRNA expression in adenomas *versus* adenocarcinomas (Figure 4) and scatter plots of mRNA expression versus DNA copy number ratio (Figure 5) are shown.

Of these seven candidate genes, 6 map within the SROs determined by STAC analysis. The seventh gene (*AURKA*) maps approximately 400kb proximal to SRO2 at 55.6Mb (20q13.31). *C20orf24* maps within SRO1 at 35.9Mb (20q11.23), *RNPC1* and *THIL* map within SRO2 at position 56.7 and 58.3Mb, respectively (20q13.32), and genes *ADMR1, C20orf20* and *TCFL5* map within SR03, the first at 61.6 and the other two at 62.2Mb (20q13.33).

Stepwise linear discriminant analysis with leave one out cross validation showed that mRNA expression levels of two out of the seven candidate genes, i.e. *RNPC1* and *TCFL5*, allowed to correctly classify 88.2% of the cases (60/68) as adenomas or carcinomas (Figure 6 and Table 3).

**Table 3. Results of stepwise linear discriminant analysis with leave one out cross validation of the seven candidate genes. From 68 tumors in total, 60 were correctly classified (88.2%), using expression levels of RNPC1 and TCFL5 only.**

| | | | **Predicted Group Membership** | | |
|---|---|---|---|---|---|
| **Lesion** | | | **Adenoma** | **Carcinoma** | **Total** |
| **Original** | **Count** | **Adenoma** | 35 | 2 | 37 |
| | | **Carcinoma** | 6 | 25 | 31 |
| | **%** | **Adenoma** | 94.6 | 5.4 | 100.0 |
| | | **Carcinoma** | 19.4 | 80.6 | 100.0 |

### Example 5 - Confirmation of differential expression by qRT-PCR

qRT-PCR was performed on a sub-sample (n = 30) of frozen tumors (15 adenomas and 15 adenocarcinomas) to confirm the expression levels of six of the seven genes identified.

Adenocarcinomas showed higher expression of all 6 genes compared to adenomas and tumors with 20q gain (4 adenomas and 8 adenocarcinomas) showed higher expression compared to tumors without 20q gain (11 adenomas and 7 adenocarcinomas). Table 4 shows the fold changes observed between either adenocarcinomas *versus* adenomas or tumors with 20q gain *versus* tumors without 20q gain, by microarrays and by qRT-PCR.

**Table 4. Expression fold-changes and range of expression levels (log₂ ratio) determined by expression microarray and by qRT-PCR, comparing either adenocarcinomas versus adenomas (Ca/Ad) or tumors with 20q gain versus tumors without 20q gain (20q gain/non 20q gain); nd, not determined.**

| **Gene** | **Comparison** | **Array fold change** | **qRT-PCR fold change** | **Microarray Expression range ^{a}** | **qRT-PCR Expression range ^{a}** |
|---|---|---|---|---|---|
| *C20orf24* | Ca/Ad 20q gain/non gain | 1.54 1.68 | 1.78 3.99 | [-0.45,1.60]/[-0.71,0.71] [-0.17,1.60]/[-0.71,0.37] | [1.84,6.08]/[-0.26,4.81] [-0.26,6.08]/[1.85,4.95] |
| *AURKA* | Ca/Ad 20q gain/non gain | 1.91 1.55 | 3.39 4.53 | [-2.01,0.17]/[-2.26,-1.11] [-2.11,0.17]/[-2.26,-0.48] | [-1.78,6.06]/[-0.64,3.72] [1.03,6.06]/[-1.78,3.99] |
| *RNPC1* | Ca/Ad 20q gain/non gain | 1.74 1.58 | nd nd | [-1.61,1.22]/[-1.80,-0.41] -1.71-1.22/-1.80--0.01 | nd nd |
| *TH1L* | Ca/Ad 20q gain/non gain | 1.52 1.59 | 4.98 6.4 | [-0.77,1.39]/[-1.06,-0.15] [-0.59,1.39]/[-1.06,0.10] | [-1.97,6.27]/[-3.57,3.72] [-3.57,6.27]/[-3.57,3.72] |
| *ADRM1* | Ca/Ad 20q gain/non gain | 1.45 1.38 | 1.46 2.58 | [-0.62, 0.79]/[-1.14,0.02] [-0.69,0.78]/[-1.14,0.36] | [-0.30,5.58]/[-1.29,5.34] [-1.29,5.58]/[-0.30,5.34] |
| *C20orf20* | Ca/Ad 20q gain/non gain | 1.36 1.34 | 3.08 3.57 | [-0.94,0.49]/[-1.31,-0.59] [-0.89,0.49]/[-1.31,-0.36] | [-1.32,2.07]/[-2.79,0.14] [-1.16,2.06]/[-2.79,0.35] |
| TCFL5 | Ca/Ad 20q gain/non gain | 2.2 2.02 | 3.54 3.54 | [-2.14,0.83]/[-2.73,-1.17] [-2.31,0.83]/[-2.73,-0.93] | [2.07,6.94]/[-1.28,4.21] [-1.28,6.94]/[1.99,4.41] |

| | | | | | |
|---|---|---|---|---|---|
| ^{a} Log₂ ratio. | | | | | |

*In situ* confirmation of AURKA expression by immunohistochemistry on TMAs yielded higher expression of AURKA in adenocarcinomas as compared to adenomas (p = 0.01) (Table 5) as well as a significant positive correlation with the mRNA expression levels (p = 0.01) (Figures 7 and 8). Validation of other genes was hampered by the absence of adequate antibodies.

**Table 5. AURKA protein expression in colorectal adenocarcinomas versus adenomas by immunohistochemistry on TMAs.**

| | | **AURKA staining** | | | | |
|---|---|---|---|---|---|---|
| | | **Negative** | **Weak** | **Strong** | **Total** | **p value^{a}** |
| **Lesion** | **Adenoma** | 12 | 12 | 1 | 25 | |
| | **Carcinoma** | 4 | 9 | 6 | 19 | |
| **Total** | | 16 | 21 | 7 | 44 | 0.01 |

| | | | | | | |
|---|---|---|---|---|---|---|
| ^{a} Cochran-Armitage test | | | | | | |

### Example 6 - Evaluation of results

One of the most frequent chromosomal aberrations observed in CRC is a gain of the long arm of chromosome 20. In order to try to identify these putative oncogenes, a series of colorectal tumors, both adenomas and carcinomas, was analyzed at the DNA and RNA levels.

In this study, it was confirmed that chromosome 20 is the most frequently altered chromosome in the progressed adenomas and adenocarcinomas (in more than 60% of cases). In non-progressed adenomas, gains of 20q were detected in less than 20%, supporting a role of 20q gain in colorectal adenoma to adenocarcinoma progression consistent with earlier observations (Hermsen, M. et al., *supra*). Narrowing down the gained region by array CGH across all tumors analyzed yielded three smallest regions of overlap: SR01 at 20q11.22-q11.23 (32-36 Mb), SRO2 at 20q13.32-q13.33 (56-59 Mb), and SRO3 at 20q13.33 (61-64 Mb).

Looking at the same expression data from a different angle, i.e. comparing the expression of tumors with and without 20q gain, it was aimed to find genes with a dosage effect on expression. Genome-wide, expression of 127 out of 931 genes was related to 20q gain, 21 of which are located at chromosome 20q itself. Although chromosome 20 has a high gene density, and copy number gains of the long arm are very frequent, certainly not all genes mapping at the gained regions are recurrently over-expressed. Two hundred and nine genes are mapped to the SROs defined here, but only 21 genes are recurrently up-regulated in association with 20q gain.

Nine genes overlapped between the 14 adenoma *versus* adenocarcinoma genes and the 21 genes associated with either or not 20q gain, namely *TPX2, C20orf24, AURKA, RNPC1, THIL, ADRM1, C20orf20, TCFL5* and *C20orf11*.

In the third approach, integration of DNA copy number changes and gene expression in the present study demonstrated that throughout the genome 507 genes showed a statistically significant association between DNA copy number and mRNA expression status, both for amplified/up-regulated and deleted/down-regulated genes, 120 of these being located on chromosome 20q. From these 120 genes, 17 overlapped with the 20q gain associated list, and 11 overlapped with the adenoma and adenocarcinoma *versus* adenocarcinoma list. Overlapping these three approaches (expression in adenomas *versus* adenocarcinomas, expression *versus* 20q gain, and genome wide expression *versus* whole genome copy-number changes) showed that seven genes are consistently significant (Figure 4), namely *C20orf24, AURKA, RNPC1, THIL, ADRM1, C20orf20* and *TCFL5*.

In addition to the already stringent data analysis, a permutation analysis was performed, comparing the differential expression of the seven 20q genes with the expression of over 50.000 random subsets out of genes 7946 in silent DNA regions (2q, 3, 5,10p, 11, 16, 21, 22). For each random subset, the Wilcoxon scores of the seven most differentially expressed (adenoma *versus* adenocarcinoma) genes were selected. The seven genes on 20q showed a significantly higher expression in adenocarcinomas *versus* adenomas compared to the best performing combination from the permutation test (p = 0.001), underlining that the copy number based discovery of putative oncogenes did not yield random differentially expressed genes. The fact these over-expressed putative oncogenes on 20q actually resulted in biologically active components, i.e. proteins, in the tumour cells was demonstrated by immunohistochemistry on TMA for AURKA. For the other candidates, antibodies did not perform adequately in the tissue samples or were not available at all.

The function of these genes include a function as transcription factors, like *TCFL5* (Siep, M. et al. (2004) Nucleic Acids Res. 32, 6425-6436), or factors being involved in transcriptional regulation, like *C20orf20* (Cai, Y. et al. (2003) J. Biol. Chem. 278, 42733-42736). *THIL* product is involved in regulation of A-Raf kinase (Liu, W. et al. (2004) J. Biol. Chem. 279, 10167-10175). *ADRM1* encodes for a putative cell adhesion molecule that recently was shown to be component of the 26S proteosome (Jorgensen, J.P. et al. (2006) J. Mol. Biol. 360, 1043-1052). *RNPC1* product is predicted to bind to RNA, based on sequence motifs and *C20orf24* interacts with Rab-*5.. AURKA* has been well characterized and is involved in cell cycle regulation. It has been shown to be amplified in CRC (Bischoff, J.R. et al. (1998) EMBO J. 17, 3052-3065) and its over-expression induces centrosome amplification, aneuploidy and transformation *in vitro* (Zhou, H. et al. (1998) Nat. Genet. 20, 189-193). Moreover, inhibiting *AURKA* by RNA interference lead to growth suppression of human pancreatic cancer cells (Hata, T. et al. (2005) Cancer Res. 65, 2899-2905). Knocking down *TCFL5* resulted in suppression of the number of multicellular HT29 tumour spheroids, supporting its role in cancer development (Dardousis, K. et al. (2007) Mol. Ther. 15, 94-102).

In summary, the above provided experimental results demonstrated the involvement of three SROs in the 20q amplicon in CRC and showed strong DNA copy number / mRNA expression associations for seven genes in these areas. In addition significant differences between colorectal adenomas and adenocarcinomas were shown at the DNA, mRNA and, for a one of the genes, at the protein level, supporting an important role as oncogenes in colorectal adenoma to adenocarcinoma progression. Furthermore, it was demonstrated that the expression levels of the marker genes of the present invention, in particular the expression levels of *RNPC1* and *TCFL5* allowed discriminating adenomas from adenocarcinomas with high accuracy.

## Claims

1. An *in vitro* method for diagnosing in a subject an adenocarcinoma associated with a chromosomal aberration on chromosome 20q, the method comprising the steps of:
(a) detecting in a test sample obtained from the subject the expression level(s) of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *THIL* (Genbank accession # NM_016397); and
(b) comparing the expression level(s) obtained in step (a) to a control level,
wherein an elevated expression level of any one of the marker genes in the test sample as compared to the control level is indicative of an adenocarcinoma associated with a chromosomal aberration on chromosome 20q in the subject.

2. The method of claim 1, for the further use of diagnosing a predisposition for developing an adenocarcinoma, a progression of an adenoma to an
adenocarcinoma or a predisposition for a progression of an adenoma to an adenocarcinoma, the adenocarinoma being associated with a chromosomal aberration on chromosome 20q.

3. The method of claim 1 or 2, wherein the chromosomal aberration on chromosome 20q is an aberration at position 20q11.22-20q11.23 and/or at position 20q13.31-20q13.33.

4. The method of any one of claims 1 to 3, wherein the chromosomal aberration is a chromosomal gain.

5. The method of any one of claims 1 to 4, wherein the expression levels of at least the marker genes *RNPC1* (Genbank accession # NM_017495) and *TCFL5* (Genbank accession # NM_006602) are detected, wherein elevated expression levels of both said marker genes in the test sample as compared to the control level, are indicative of an adenocarcinoma, a predisposition for developing an adenocarcinoma, a progression of an adenoma to an adenocarcinoma or a predisposition for a progression of an adenoma to an adenocarcinoma, the adenocarcinoma being associated with a chromosomal aberration on chromosome 20q in the subject.

6. The method of claim 1 to 5, wherein the expression level(s) of the marker gene(s) is (are) determined by any one or more of the methods selected from the group consisting of:
(a) detecting a mRNA encoded by the marker gene(s);
(b) detecting a protein encoded by the marker gene(s); and
(c) detecting a biological activity of a protein encoded by the marker gene(s).

7. The method of any one of claims 1 to 6, further comprising a step (c) of detecting a chromosomal aberration on chromosome 20q, preferably by
comparative genomic hybridization (CGH), PCR detection or multiplex ligation-dependent probe amplification (MPLA).

8. A *in vitro* method for diagnosing in a subject an adenocarcinoma, the method comprising:
(a) detecting in a test sample obtained from the subject a chromosomal gain on chromosome 20q; and in case a chromosomal gain is detected on chromosome 20q further comprising the steps of
(b) detecting in said sample the expression level(s) of at least one of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *THIL* (Genbank accession # NM_016397); and
(c) comparing the expression level(s) obtained in step (b) to a control level, wherein an elevated expression level of any one of the marker genes in the test sample as compared to the control level is indicative of an adenocarcinoma.

9. The method of claim 8, wherein the detection of a chromosomal gain on
chromosome 20q is performed by comparative genomic hybridization (CGH), PCR detection or multiplex ligation-dependent probe amplification (MPLA).

10. A kit for diagnosing adenocarcinoma comprising means for detecting the expression of at least one of the marker genes *RNPC1* (Genbank accession #
NM_017495), *TCFL5* (Genbank accession # NM_006602), *C20orf24* (Genbank accession # NM_018840), *AURKA*/*STK6* (Genbank accession # NM_003600),
*C20orf20* (Genbank accession # NM_018270), *ADRM1* (Genbank accession # NM_007002), and *THIL* (Genbank accession # NM_016397).

11. The kit of claim 10, further comprising means for detecting a chromosomal aberration on chromosome 20q.

12. A method of identifying an agent for preventing and/or treating adenocarcinoma, the method comprising the steps of:
(a) contacting a test agent with one or more cells expressing any one or more of the marker genes *RNPC1* (Genbank accession # NM_017495), *TCFL5* (Genbank accession # NM_006602), *C20orf24* (Genbank accession # NM_018840), *AURKA*/*STK6* (Genbank accession # NM_003600), *C20orf20* (Genbank accession # NM_018270), *ADRM1* (Genbank accession # NM_007002), and *THIL* (Genbank accession # NM_016397);
(b) detecting the expression level(s) of the one or more marker genes; and
(c) selecting a test agent that reduces the expression level(s) of any one or more of the marker gene as compared to that (those) detected in the absence of the test agent.

13. A pharmaceutical composition comprising any one or more agents selected from the group consisting of: an antisense nucleic acid construct, an siRNA, a riboyzme or an antibody directed against or a dominant negative polypeptide variant of any one of the marker genes *RNPC1* (Genbank accession #
NM_017495), *TCFL5* (Genbank accession # NM_006602), *C20orf24* (Genbank accession # NM_018840), *AURKA*/*STK6* (Genbank accession # NM_003600),
*C20orf20* (Genbank accession # NM_018270), *ADRM1* (Genbank accession # NM_007002), and *THIL* (Genbank accession # NM_016397).

14. The pharmaceutical composition of claim 13 for the prevention and/or treatment of an adenocarcinoma.

15. Use of an antisense nucleic acid construct, an siRNA, a riboyzme or an antibody directed against or a dominant negative polypeptide variant of any one of the marker genes *RNPC1* (Genbank accession # NM_017495), *TCFL5* (Genbank
accession # NM_006602), *C20orf24* (Genbank accession # NM_018840),
*AURKA*/*STK6* (Genbank accession # NM_003600), *C20orf20* (Genbank accession # NM_018270), *ADRM1* (Genbank accession # NM_007002), and
*THIL* (Genbank accession # NM_016397) for the preparation of a pharmaceutical composition for the prevention and/or treatment of an adenocarcinoma.
